# EUROPÄISCHE PATENTSCHRIFT

(11) **EP 3 526 735 B1**
(45) Veröffentlichungstag und Bekanntmachung des Hinweises auf die Patenterteilung: **05.08.2020**
(21) Anmeldenummer: 17790964.5
(22) Anmeldetag: 02.10.2017
(51) Int. Cl.: G06M 1/08, G06M 1/27

(54) **GRENZWERTDETEKTIONSVORRICHTUNG**
LIMITING VALUE DETECTION DEVICE
DISPOSITIF DE DÉTECTION D'UNE VALEUR LIMITE

(30) Priorität: 14.10.2016 DE 102016220111
(43) Veröffentlichungstag der Anmeldung: 21.08.2019
(73) Patentinhaber: Hahn-Schickard-Gesellschaft für angewandte Forschung e.V., 78052 Villingen-Schwenningen (DE)
(72) Erfinder: SPIETH, Sven, 78083 Dauchingen (DE)
(74) Vertreter: Stöckeler, Ferdinand
(86) Internationale Anmeldenummer: PCT/EP2017/074948
(87) Internationale Veröffentlichungsnummer: WO 2018/069079

(56) Entgegenhaltungen:
- EP-A1- 0 979 658
- EP-A2- 1 844 838
- DE-A1-102012 200 740
- DE-U1-202014 005 485

## Beschreibung

Die Erfindung betrifft eine Vorrichtung mit den Merkmalen von Anspruch 1.

Die erfindungsgemäße Vorrichtung kann verschiedenste Grenzwertereignisse detektieren. Solche Grenzwertereignisse definieren sich dadurch, dass ein vordefinierter Schwellenwert unter- bzw. überschritten wird. Hierbei kann es sich beispielsweise um Schwellenwerte eines Drucks, einer Temperatur, einer Beschleunigung, einer mechanischen Kraft, und dergleichen handeln.

Das Erfassen und der Nachweis solcher Grenzwertüber- bzw. -unterschreitungen in einem betrachteten Zeitraum kann bei verschiedenen industriellen Prozessen von Bedeutung sein. Eines von unzähligen Beispielen betrifft beispielsweise kritische Temperaturbelastungen während der Produktherstellung, in der Logistikkette, während der Produktnutzung oder allgemein bei temperaturbehafteten Prozessen an einem Produkt.

Auch Beschleunigungs-Grenzwertüberschreitungen, z.B. in Fallsensoren bei Smartphones, oder Druck-Grenzwertüberschreitungen, z.B. in Gasflaschen, sind heutzutage von großem Interesse in der Industrie. Oftmals ist es hierbei interessant zu erfahren, wie oft ein vordefinierter Grenzwert unter- bzw. überschritten wurde.

Die WO 2005/081730 A2 beschreibt beispielsweise eine Vorrichtung zum Zählen der Anzahl durchgeführter Sterilisations- bzw. Nutzungszyklen. Diese Vorrichtung ist in Feinwerktechnik ausgeführt, was sich deutlich von den erhöhten Anforderungen an die Präzision von in Mikrosystemtechnik gefertigten Bauteilen unterscheidet. Die bekannte Vorrichtung weist ein Zahnrad oder eine Zahnstange auf, die jeweils mittels eines Kolbens betätigt werden. Die Vorrichtung weist Steckverbinder auf, mit denen die Vorrichtung in eine externe Apparatur eingesteckt werden kann. Im Bereich dieser Stecker ist außerdem ein Ende des Kolbens angeordnet. Wenn diese Vorrichtung in die externe Apparatur eingesteckt wird, stößt der Kolben an die externe Apparatur an und wird durch die Einsteckbewegung verschoben. Der Kolben betätigt dadurch das Zahnrad bzw. die Zahnstange und schiebt es/sie um einen Zahn weiter. Ein optisches Anzeigemittel in Form einer mechanischen Zahlenskala wird hierbei eingesetzt, um dem Nutzer die Anzahl der Nutzungszyklen anzuzeigen.

Die DE 10 2005 054 546 A1 beschreibt ein System zum Erfassen der Durchführung eines Behandlungsprozesses an einem Gegenstand. Die hierin offenbarte Vorrichtung weist einen einzelnen Arretierhaken und einen thermisch verformbaren Bimetallstreifen auf, der in einem unbetätigten Zustand einen Zählerschalter betätigt. Mit ansteigender Temperatur wird der Bimetallstreifen in Richtung des Arretierhakens ausgelenkt und er hängt sich dabei in den Arretierhaken ein. Hierbei betätigt der Bimetallstreifen einen auf dem Arretierhaken angeordneten Sicherheitsschalter. Beim Auslesen der Vorrichtung mittels RFID wird eine Rückstelleinrichtung betätigt, die den Arretierhaken zurückschwenkt, so dass der Bimetallstreifen zurück auf den Zählerschalter fällt. Dies geschieht aber freilich nur dann, wenn die Temperatur bereits wieder soweit abgesunken ist, dass der Bimetallstreifen auch schon wieder in seine unausgelenkte Position zurückdrängt. Das viel größere Problem bei dieser Vorrichtung besteht jedoch darin, dass sich der Mechanismus nicht selbst zurückstellt, d.h. es muss nach jedem Sterilisationsvorgang eine Auslesung mittels RFID erfolgen, damit der Rückstellmechanismus betätigt wird. Wird aber beispielsweise zwischen zwei Operationseinsätzen ein Auslesen der Vorrichtung vergessen, kann im Nachhinein nicht mehr überprüft werden, ob zwischen den beiden OPs tatsächlich eine Sterilisation erfolgt ist, oder ob der Bimetallstreifen immer noch wegen der vor der ersten OP erfolgten Sterilisation an dem Arretierhaken hängt.

In [1] ist ein mikromechanischer Grenzwertzähler zum Aufzählen von bis zu fünf Beschleunigungsgrenzwertereignissen beschrieben. Ein Biegebalken mit einer daran angeordneten Masse ist einseitig eingespannt und sein offenes Ende ist in einem kammähnlichen Element mit Lücken angeordnet. Bei einer Beschleunigung wird die am Biegebalken angeordnete Masse in Abhängigkeit vom Betrag der Beschleunigung ausgelenkt, so dass das offene Ende des Biegebalkens in einer der Lücken des kammähnlichen Elements zu liegen kommt. Diese Vorrichtung ist ein passives System, das keine zusätzlichen Antriebselemente benötigt. Jedoch bietet diese Vorrichtung keinen Rückstellmechanismus, d.h. sofern der Biegebalken einmal ausgelenkt wurde, stellt er sich nicht automatisch wieder in die Ausgangslage zurück. Ganz abgesehen davon reagiert die Masse nur auf den Betrag der Beschleunigung, nicht jedoch auf die Anzahl der Beschleunigungsvorgänge, d.h. bei einem einzigen aber starken Beschleunigungsvorgang kann das offene Ende des Biegebalkens um zwei oder mehrere Lücken weiter springen. Mit dieser Vorrichtung kann demnach nicht sichergestellt werden, dass pro Grenzwertereignis lediglich eine Raste weitergesprungen wird. Damit kann nachträglich nicht mehr differenziert werden, ob eine sehr starke Beschleunigung oder mehrere schwache Beschleunigungen stattgefunden haben.

Ein ähnliches System ist in [2] beschrieben. Eine seismische Masse ist schwimmend an zwei Federn gelagert. Die seismische Masse ist innerhalb eines stationären Ratschenelements angeordnet. Das Ratschenelement weist Vorsprünge auf und die seismische Masse weist Haken auf, die in die Vorsprünge eingreifen können. Diese Vorrichtung weist im Wesentlichen dieselben Vor- und Nachteile wie das in [1] beschriebene System auf. Diese Vorrichtung ist ebenfalls ein passives System, das keine zusätzlichen Antriebselemente benötigt. Jedoch bietet auch diese Vorrichtung keinen Rückstellmechanismus, d.h. sofern die seismische Masse einmal ausgelenkt wurde, stellt sie sich nicht automatisch wieder in die Ausgangslage zurück. Ganz abgesehen davon reagiert auch hier die seismische Masse nur auf den Betrag der Beschleunigung, nicht jedoch auf die Anzahl der Beschleunigungsvorgänge, d.h. bei einem einzigen aber starken Beschleunigungsvorgang kann die seismische Masse um zwei oder mehrere Vorsprünge weiter springen. Auch mit dieser Vorrichtung kann demnach nicht sichergestellt werden, dass pro Grenzwertereignis lediglich eine Raste weitergesprungen wird. Damit kann nachträglich nicht mehr differenziert werden, ob eine sehr starke Beschleunigung oder mehrere schwache Beschleunigungen stattgefunden haben.

Der nächstliegende Stand der Technik, EP 0 979 658 A1 beschreibt eine Röhre, wobei sich innerhalb dieser Röhre ein beweglicher Körper befindet. Die EP 0 979 658 A1 erwähnt mehrfach in der Beschreibung, dass eine deutlich ablesbare Skala vorhanden sein soll. Eine solche deutlich ablesbare Skala kann jedoch nicht im Mikrometerbereich gefertigt sein, da sie ansonsten ohne Hilfsmittel nicht lesbar ist. Außerdem erwähnt die EP 0 979 658 A1, dass die Röhre typischerweise 5 cm lang ist und einen Durchmesser von 10 mm aufweist.

Die EP 1 844 838 A2 beschreibt ein RFID-gestütztes System zum Messen von Temperaturen eines Filterelements. Hierfür sind ein Temperaturwandler und eine Kommunikationsvorrichtung derart miteinander gekoppelt, um im laufenden Betrieb die Temperatur des Filterelements zu messen und per Funk zu übertragen.

Es ist eine Aufgabe der vorliegenden Erfindung, eine Vorrichtung bereitzustellen, die geeignet ist, um Grenzwertereignisse sowohl zu erfassen als auch die Anzahl der Grenzwertunter- bzw. -überschreitungen zu bestimmen, jedoch unter Umgehung der im Stand der Technik genannten Probleme.

Diese Aufgabe wird erfindungsgemäß durch eine Vorrichtung mit den Merkmalen von Anspruch 1 gelöst.

Die erfindungsgemäße Vorrichtung weist hierfür, unter anderem, einen Rastmechanismus auf. Der Rastmechanismus weist ein Rastenelement mit mindestens zwei Rasten auf. Außerdem weist der Rastmechanismus eine Klinke auf, die ausgebildet ist, um in einen Rastenzwischenraum zwischen zwei Rasten einzugreifen. Der Rastenzwischenraum ist die Lücke zwischen zwei Rasten. Das Rastenelement ist in einer ersten Richtung relativ zu der Klinke bewegbar. Diese erste Richtung wird daher auch als Freilaufrichtung bezeichnet. Eine Bewegung des Rastenelements relativ zu der Klinke in einer zweiten Richtung ist hingegen mittels der Klinke blockierbar. Diese zweite Richtung wird daher auch als Sperrrichtung bezeichnet. Das Rastenelement kann sich also in Freilaufrichtung relativ zu der Klinke bewegen. Eine Bewegung des Rastenelements in Sperrrichtung wird also durch die Klinke verhindert. Die erfindungsgemäße Vorrichtung weist ferner ein auslenkbares Betätigungsmittel auf, das ausgebildet ist, um mittels einer Auslenkung das Rastenelement und die Klinke relativ zueinander rastenweise in Freilaufrichtung zu bewegen. Das Betätigungsmittel betätigt beispielsweise das Rastenelement oder die Klinke, um eine Relativbewegung zwischen dem Rastenelement und der Klinke zu ermöglichen. Erfindungsgemäß findet diese Relativbewegung aber nur rastenweise statt. Das heißt, das Rastenelement und die Klinke werden mit jeder Auslenkung des Betätigungsmittels nur um eine Raste weiterbewegt. Oder anders ausgedrückt, das Rastenelement und die Klinke werden mit jeder Auslenkung des Betätigungsmittels relativ zueinander derart bewegt, dass sich das Rastenelement relativ zu der Klinke nur schrittweise um jeweils eine Raste pro Betätigung weiterbewegt. Das Betätigungsmittel schiebt dabei das Rastenelement relativ zu der Klinke bei jeder Grenzwertüberschreitung um jeweils eine Raste weiter. Hierfür kann sich das Betätigungsmittel nach einer erfolgten Über- bzw. Unterschreitung des vordefinierten Grenzwerts wieder in seine Ausgangslage zurückstellen. Somit kann also das Eintreten eines Grenzwertereignisses mehrfach detektiert werden. Das Betätigungsmittel ist hierbei das Bauteil, das sensitiv auf die zu messende Größe reagiert. Das heißt, das Betätigungsmittel kann beispielsweise ansprechend auf eine Kraft, eine Temperatur, einen Druck, einem elektrischen Strom, und dergleichen derart ausgelenkt werden, dass es das Rastenelement oder die Klinke betätigt und diese relativ zueinander um jeweils eine Raste bewegt, wenn ein vordefinierter Schwellenwert der zu messenden Größe unter- bzw. überschritten ist. Die erfindungsgemäße Vorrichtung weist außerdem ein elektrisches Bauelement auf, das ausgebildet ist, um seine elektrische Eigenschaft in Abhängigkeit von der rastenweisen Bewegung des Rastenelements relativ zu der Klinke zu verändern. So kann das elektrische Bauelement seine elektrische Eigenschaft beispielsweise jedes Mal, wenn sich das Rastenelement relativ zu der Klinke um eine Raste weiterbewegt hat, ändern. Dabei kann die variable elektrische Eigenschaft des elektrischen Bauelements bei jeder einzelnen Position des Rastenelements relativ zu der Klinke einen spezifischen Wert annehmen. Beispielsweise kann es sich bei dem elektrischen Bauelement um einen variablen Widerstand, einen Kondensator oder aber auch um eine Spule handeln, dessen jeweiliger Betrag (Widerstand, Kapazität, Induktivität) sich mit jeder rastenweisen Bewegung des Rastenelements relativ zu der Klinke ändert. Im Umkehrschluss ist also jeder einzelne Widerstands-, Kapazitäts- oder Induktivitätswert für jeweils eine bestimmte Position des Rastenelements relativ zu der Klinke charakteristisch. Somit kann von dem aktuellen Messwert des elektrischen Bauelements auf die aktuelle Position des Rastenelements relativ zu der Klinke rückgeschlossen werden. Dementsprechend kann von dieser ermittelten Position des Rastenelements relativ zu der Klinke wiederum darauf geschlossen werden, um wie viele Rasten sich das Rästenelement relativ zu der Klinke bereits (ausgehend von einer Ausgangsposition) weiterbewegt hat, d.h. wie viele Grenzwertüber- bzw. - unterschreitungen bereits stattgefunden haben. Somit kann also nicht nur das Eintreten eines Grenzwertereignisses detektiert werden, sondern die erfindungsgemäße Vorrichtung ist außerdem dazu in der Lage, die Anzahl der Grenzwertüberschreitungen mittels des elektrischen Bauelements zu erfassen und gegebenenfalls zu speichern.

Erfindungsgemäß ist der Rastmechanismus als ein Mikrosystem (MEMS: microelectromechanical system) ausgeführt. Ein MEMS unterscheidet sich hinsichtlich Aufbau und Anforderung an dessen Herstellung deutlich von feinwerktechnischen Aufbauten. Während feinwerktechnische Strukturen, z.B. Zahnräder für Uhrwerke, meist gestanzt oder gelegentlich auch gelasert werden, werden MEMS-Strukturen in der Regel unter Anwendung von Ätzprozessen hergestellt. Viele Strukturen, die in Feinwerktechnik herstellbar sind, können in MEMS-Technik nur sehr schwer oder gar nicht realisiert werden. Die Herstellung des Rastmechanismus als ein MEMS bringt jedoch den entscheidenden Vorteil, dass der Rastmechanismus sehr kompakt und platzsparend wird. Insbesondere im Vergleich zu den vorgenannten Feinwerktechnik-Strukturen sind MEMS-Strukturen oftmals um Faktoren mehrerer Zehnerpotenzen kleiner.

Ferner weist die erfindungsgemäße Vorrichtung ein Substrat auf, auf dem der Rastmechanismus als ein MEMS bereitgestellt ist. Einerseits kann hierbei eine Auslenkung des Betätigungsmittels in einer Ebene horizontal zu der Substratebene geschehen. Als Substratebene wird die Ebene bezeichnet, die von den lateralen Außenkanten des Substrats begrenzt bzw. aufgespannt wird. Bei einem Wafer beispielsweise ist die Substratebene etwa gleichzusetzen mit dem an sich flachen Wafer selbst. Eine Bewegung innerhalb einer Ebene parallel zu der Substratebene kann beispielsweise eine Bewegung in oder auf dem Substrat sein, sofern sich das Bewegungsmittel parallel zu der Substratebene bewegt.

Alternativ dazu wäre es denkbar, dass eine Auslenkung des Betätigungsmittels senkrecht zu der Substratebene geschieht, und wobei die Vorrichtung ferner eine Umlenkvorrichtung aufweist mittels der die senkrecht zu der Substratebene gerichtete Auslenkbewegung des Betätigungsmittels in eine horizontal zur Substratebene gerichtete Bewegung umlenkbar ist. Eine Bewegung senkrecht zu der Substratebene wäre beispielsweise eine Bewegung des Betätigungsmittels aus der Substratebene heraus, d.h. das Betätigungsmittel würde sich z.B. senkrecht von dem Substrat weg bewegen. Eine entsprechende Umlenkvorrichtung kann beispielsweise in Form von Zahnrädern, insbesondere Kegel- oder Schneckenrädern, bereitgestellt werden. Es wäre aber auch denkbar, dass die Umlenkvorrichtung ein erstes und ein zweites Umlenkmittel aufweist, wobei das erste Umlenkmittel eine schräge Fläche aufweist und das zweite Umlenkmittel mit dieser schrägen Fläche in Kontakt ist. Wenn das zweite Umlenkmittel nun Druck auf die schräge Fläche ausübt, dann bewegt sich das erste Umlenkmittel in eine Richtung, die schräg zu der Bewegungsrichtung des zweiten Umlenkmittels ist. Beispielsweise kann bei einer schrägen Fläche mit einem Winkel von 45° eine Umlenkung von einer horizontalen in eine vertikale Bewegung realisiert werden. Die Betätigung der Klinke bzw. des Rastenelements geschieht in diesem Fall nicht unmittelbar durch das Betätigungsmittel sondern mittelbar mittels der dazwischen angeordneten Umlenkvorrichtung. Das heißt, das Betätigungsmittel betätigt die Umlenkvorrichtung (senkrecht zur Substratebene) und die Umlenkvorrichtung betätigt die Klinke bzw. das Rastenelement (horizontal zur Substratebene).

Gemäß einer Ausführungsform sind die Rasten entlang des Rastenelements in Freilaufrichtung hintereinander angeordnet, sodass die Klinke bei der rastenweisen Bewegung nacheinander von einem Rastenzwischenraum in den jeweils nächsten benachbarten Rastenzwischenraum eingreift. Dies unterscheidet die erfindungsgemäße Vorrichtung von anderen Vorrichtungen, die nur ein Rastenelement mit einer einzelnen Raste und einer Klinke aufweisen. Während bei derartigen Systemen nämlich nach einmaliger Betätigung zwingend ein Rückstellmechanismus benötigt wird, kann bei der erfindungsgemäßen Vorrichtung das Rastenelement relativ zu der Klinke mehrfach bewegt werden.

Es ist außerdem vorstellbar, dass das Rastenelement ein frei drehbares Zahnrad ist, bei dem die Rasten in Form einer radial außenseitig oder radial innenseitig an dem Zahnrad angeordneten Verzahnung ausgebildet sind. Ein solches Zahnrad ist auch in MEMS-Technik relativ einfach fertigbar. Außerdem bietet die Ausführung des Rastenelements als ein Zahnrad den Vorteil, dass das Zahnrad sozusagen endlos gegenüber der Klinke (rastenweise) weiterbewegbar ist. Es wäre aber auch denkbar, dass ein Endanschlag vorgesehen ist, der die Anzahl der rastenweisen Bewegungen begrenzt. Beispielsweise könnte der Endanschlag nach einer vollen Umdrehung des Zahnrads eine weitere Drehung des Zahnrads begrenzen. Somit kann vermieden werden, dass eine mit dem Zahnrad verbundene Zählerschaltung nach einer erfolgten 360° Drehung des Zahnrads genullt wird. Dies hätte also den Vorteil, dass z.B. bei einem Zahnrad, das ja wie zuvor erwähnt theoretisch unendlich weiterdrehbar ist, der Endanschlag derart ausgebildet ist, dass sich das Zahnrad nur höchstens ein einziges Mal um 360° dreht. So kann sichergestellt werden, dass es nicht zu Überschneidungen beim Auslesen des Zählerstands kommt. Das heißt, jede Position des Zahnrads kann mittels des Endanschlags bei 360° nur ein einziges Mal eingenommen werden. Somit ist ein mit einer bestimmten Position des Zahnrads verknüpfter Wert des elektrischen Bauelements ebenfalls nur einmal produzierbar und somit eindeutig. Es kann also nicht zu Doppeldeutigkeiten kommen. Ein Endanschlag kann aber auch bei Zahnstangen und dergleichen eingesetzt werden, um eine bestimmte Anzahl von rastenweisen Bewegungen nicht zu überschreiten.

Ebenso wäre es denkbar, dass das durch den Antriebsmechanismus angetriebene Rastenelement, in diesem Fall das Zahnrad, wiederum in weitere Elemente eingreift, z.B. weitere Zahnräder, so dass sich ein Getriebe ergibt. Bei entsprechender Verzahnung (Untersetzung) kann das Rastenelement so um mehr als 360° gedreht werden, während sich das weitere Element, das mit dem elektrischen Bauelement verknüpft ist, dann um weniger als 360° dreht und eine eindeutige Position einnimmt.

Ebenso wäre es denkbar, dass das Rastenelement eine relativ zu der Klinke bewegbare Zahnstange ist, bei der die Rasten in Form einer an der Zahnstange angeordneten Verzahnung ausgebildet sind. Eine Zahnstange kann beispielsweise eine lineare oder aber auch eine gekrümmte Form aufweisen. Bei einer gekrümmten Form kann die Verzahnung innenseitig, d.h. zum Mittelpunkt des Krümmungsradius hin gerichtet, und/oder außenseitig, d.h. auf der dem Mittelpunkt des Krümmungsradius abgewandten Seite der Zahnstange, angeordnet sein.

Es ist vorstellbar, dass das Betätigungsmittel das Rastenelement betätigt, um das Rastenelement relativ zu der Klinke in Freilaufrichtung rastenweise um jeweils eine Raste weiterzubewegen. Wie bereits zuvor erwähnt, kann das Betätigungsmittel das Rastenelement unmittelbar oder mittelbar betätigen. Eine Betätigung des Rastenelements hat den Vorteil, dass die Klinke stationär an der Vorrichtung angeordnet werden kann, während das Rastenelement in Freilaufrichtung bewegt wird. Das rastenweise Überspringen der Klinke von einem Rastenzwischenraum zu dem nächsten Rastenzwischenraum in Freilaufrichtung kann hierbei beispielsweise durch geeignete Formgebung der Rasten und der Klinke erfolgen, sodass die Klinke beim Bewegen des Rastenelements über die Raste entlang der Rastenflanke hinweg gleitet und sich in den benachbarten Rastenzwischenraum einklinkt.

Geeigneter Weise greift das Betätigungsmittel an einer Raste des Rastenelements an, um das Rastenelement relativ zu der Klinke rastenweise weiterzubewegen. So kann das Betätigungsmittel beispielsweise an einem Zahn eines Zahnrads angreifen und das Zahnrad direkt um einen Zahn weiterbewegen. Dies ist eine relativ einfache Möglichkeit, um das Rastenelement zu betätigen, da keine weiteren Umlenkhebel, etc. benötigt werden.

Ebenfalls wäre es denkbar, dass das Rastenelement mittels eines Spannelements vorgespannt ist und das Betätigungsmittel die Klinke betätigt, wobei sich bei einer den Eingriff in einen Rastenzwischenraum lösenden Bewegung der Klinke das vorgespannte Rastenelement aufgrund der Vorspannung jeweils um eine Raste weiterbewegt bevor die Klinke wieder in einen benachbarten nächsten Rastenzwischenraum des Rastenelements eingreift. Dies wäre in etwa vergleichbar mit einer Hemmung bei einem Uhrwerk. Dies hätte den Vorteil, dass z.B. bei einem Zahnrad, das ja wie zuvor erwähnt theoretisch unendlich weiterdrehbar ist, die Vorspannung nur so weit gewählt wird, dass sich das Zahnrad nur höchstens ein einziges Mal um 360° dreht. So kann nämlich sichergestellt werden, dass es nicht zu Überschneidungen beim Auslesen des Zählerstands kommt. Das heißt, jede Position des Zahnrads kann dann aufgrund der Vorspannung nur ein einziges Mal eingenommen werden. Somit ist ein mit einer bestimmten Position des Zahnrads verknüpfter Wert des elektrischen Bauelements ebenfalls nur einmal produzierbar und somit eindeutig. Es kann also nicht zu Doppeldeutigkeiten kommen. Alternativ oder zusätzlich wäre auch hier das Vorsehen eines zuvor erwähnten Endanschlags denkbar.

Es ist vorstellbar, dass das Betätigungsmittel thermisch auslenkbar ist. Somit können thermische Grenzwertüberschreitungen gemessen werden.

Hierbei könnte das Betätigungsmittel beispielsweise ein thermischer Biegewandler sein, oder das Betätigungsmittel kann eine Formgedächtnislegierung aufweisen. Unter einem thermischen Biegewandler ist ein Bauteil zu verstehen, dass seine Form in Abhängigkeit von der Temperatur verändert. Der thermische Biegewandler kann sich beispielsweise beim Überschreiten einer Grenzwerttemperatur in eine erste Richtung verformen. Beim Unterschreiten dieser Grenzwerttemperatur kehrt der thermische Biegewandler wieder in seine Ausgangslage zurück, d.h. er verformt sich wieder in die andere Richtung. Ein thermischer Biegewandler kann auch ein im englischen Sprachraum unter dem Namen Bimorph bekanntes Bauteil sein. Ein solcher Bimorph weist zwei oder mehr aktive Bereiche auf, die getrennt voneinander betätigbar sind. Ein thermischer Bimorph beispielsweise weist zwei aktive Bereiche auf, die sich beim Überschreiten einer Grenzwerttemperatur in eine erste Richtung verformen. Beim Unterschreiten dieser Grenzwerttemperatur bewegen sich die beiden aktiven Bereiche wieder in ihre Ausgangslage, d.h. in eine entgegengesetzte zweite Richtung, zurück. Die beiden aktiven Bereiche können unterschiedliche Temperaturausdehnungskoeffizienten aufweisen. Dadurch fällt der Betrag der Verformung beider aktiver Bereiche unterschiedlich groß aus, was wiederum zu einer mechanischen Auslenkung des Bimorphs führt. Der thermische Bimorph kann sich also in Reaktion auf ein Unter- bzw. Überschreiten einer Grenzwerttemperatur in zwei Richtungen bewegen.

Alternativ oder zusätzlich kann das Betätigungsmittel mechanisch oder elektrisch auslenkbar sein. Beispielsweise kann das Betätigungsmittel mechanisch unter Anwendung bestimmter Kräfte, beispielsweise Druck, auslenkbar sein. Hierbei kann es sich beispielsweise um einen barometrischen Druck handeln, d.h. das Betätigungsmittel kann beispielsweise in Tauch-Chronographen eingesetzt werden und die Anzahl der Tauchgänge anzeigen. Das Betätigungsmittel kann aber auch beispielsweise durch Beschleunigungskräfte auslenkbar sein. Somit kann beispielsweise nachgewiesen werden, ob und wie oft ein Gerät aus einer bestimmten Höhe heruntergefallen ist, oder es kann die Anzahl von Geschwindigkeitsübertretungen in Fahrzeugen nachgewiesen werden.

Es ist denkbar, dass das Betätigungsmittel ausgebildet ist, um beim Überschreiten und/oder Unterschreiten eines vordefinierten Grenzwerts ausgelenkt zu werden, um mittels dieser Auslenkung das Rastenelement und die Klinke relativ zueinander in Freilaufrichtung zu bewegen. Ein solcher Grenzwert kann, je nachdem durch welche Kraft (z.B. thermisch, elektrisch, mechanisch) das Betätigungsmittel auslenkbar ist, beispielsweise ein vordefinierter Betrag einer Temperatur, eines Drucks, einer Beschleunigung oder anderer thermischer, elektrischer oder mechanischer Kräfte sein. Dabei kann es sich sowohl um einen oberen als auch um einen unteren Grenzwert handeln. Jedenfalls betätigt das Betätigungsmittel erfindungsgemäß die Klinke und/oder das Rastenelement nur dann, wenn die das Betätigungsmittel auslenkende Kraft den Grenzwert unter- bzw. überschreitet. Das heißt, erst beim Unterschreiten bzw. Überschreiten des vordefinierten Grenzwerts ist die Auslenkung des Betätigungsmittels ausreichend, um das Rastenelement und die Klinke relativ zueinander zu bewegen.

Gemäß einer denkbaren Ausführungsform kann das elektrische Bauelement ein Kondensator oder ein Widerstand oder eine Spule oder ein elektrooptisches Element sein. All diese elektrischen Bauelemente sind geeignet, um bereits geringste Veränderungen ihres elektrischen Verhaltens festzustellen.

Es wäre beispielsweise denkbar, dass das elektrische Bauelement ein verstellbares Glied eines RFID-Schwingkreises ist. Das elektrische Bauelement könnte also beispielsweise ein elektrischer Verbraucher mit variablem Widerstand, ein Kondensator mit variabler Kapazität oder eine Spule mit variabler Induktivität sein, wobei deren jeweilige elektrische Eigenschaft in Abhängigkeit der zu bestimmenden Größe (z.B. Temperatur, Druck, etc.) variiert. Mit Änderung der elektrischen Eigenschaft ändert sich auch die Resonanzfrequenz des gesamten RFID-Schwingkreises. Somit kann der (aktive oder passive) RFID-Schwingkreis beispielsweise mit einem RFID-Lesegerät ausgelesen werden, wobei die Frequenz des Schwingkreises ein Indikator für den aktuellen Wert des jeweiligen verstellbaren elektrischen Bauelements, und somit gleichzeitig auch ein Indikator für die aktuelle Position des Rastenelements relativ zu der Klinke sein kann.

Es ist denkbar, dass die Vorrichtung ein Substrat aufweist, auf dem der Rastmechanismus angeordnet ist, und das elektrische Bauelement zwischen dem Rastmechanismus und dem Substrat angeordnet ist. Somit kann beispielsweise ein Verschieben des Rastenelements relativ zu dem Substrat zu einer Veränderung der elektrischen Eigenschaft des elektrischen Bauelements führen. Außerdem ist diese Anordnung platzsparend, da das elektrische Bauelement beispielsweise direkt in das Substrat integriert sein kann und somit nicht neben sondern direkt unterhalb des Rastenelements angeordnet werden kann.

Gemäß einer Ausführungsform der Erfindung kann die Vorrichtung ein Substrat aufweisen, auf dem der Rastmechanismus angeordnet ist, und das elektrische Bauelement kann ein Kondensator sein, wobei eine erste Kondensatorplatte an dem Substrat und eine zweite Kondensatorplatte an dem Rastenelement und/oder an der Klinke bereitgestellt ist, und wobei sich bei einer rastenweisen Bewegung des Rastenelements und/oder der Klinke relativ zu dem Substrat die Ausrichtung der Kondensatorplatten relativ zueinander verändert, sodass sich die Kondensatorkapazität ändert.

Die erfindungsgemäße Vorrichtung kann beispielsweise als ein Sterilisationszyklenzähler ausgebildet sein, bei dem das Betätigungsmittel bei jedem Sterilisationsvorgang das Rastenelement und die Klinke in Freilaufrichtung relativ zueinander um jeweils eine Raste verschiebt.

Ausführungsbeispiele der Erfindung sind in der Zeichnung dargestellt und werden nachstehend erläutert. Es zeigen:
- Fig. 1: eine schematische Seitenansicht eines Ausführungsbeispiels einer erfindungsgemäßen Vorrichtung,
- Fig. 2: eine schematische Seitenansicht eines weiteren Ausführungsbeispiels einer erfindungsgemäßen Vorrichtung,
- Fig. 3A bis 3F: Detailansichten zur Beschreibung der Funktionsweise der erfindungsgemäßen Vorrichtung,
- Fig. 4: eine schematische Seitenansicht eines weiteren Ausführungsbeispiels einer erfindungsgemäßen Vorrichtung,
- Fig. 5: eine schematische Seitenansicht eines weiteren Ausführungsbeispiels einer erfindungsgemäßen Vorrichtung,
- Fig. 6: eine schematische Seitenansicht eines weiteren Ausführungsbeispiels einer erfindungsgemäßen Vorrichtung, bei der das Betätigungsmittel die Klinke nach dem Prinzip der Ankerhemmung betätigt,
- Fig. 7A: eine schematische Draufsicht auf ein Substrat mit einem Betätigungsmittel, um eine Bewegung des Betätigungsmittels in der bzw. horizontal zu bzw. parallel zu der Substratebene zu visualisieren,
- Fig. 7B: eine schematische Seitenansicht eines Substrats mit einem Betätigungsmittel, um eine Bewegung des Betätigungsmittels in einer Ebene senkrecht zur Substratebene zu visualisieren,
- Fig. 7C: eine schematische Seitenansicht einer erfindungsgemäßen Vorrichtung mit einer Umlenkvorrichtung,
- Fig. 7D: eine schematische Seitenansicht einer erfindungsgemäßen Vorrichtung mit einer Umlenkvorrichtung in einem ersten Zustand,
- Fig. 7E: eine schematische Seitenansicht der erfindungsgemäßen Vorrichtung aus Figur 7D in einem zweiten Zustand,
- Fig. 8: ein weiteres Ausführungsbeispiel einer erfindungsgemäßen Vorrichtung,
- Fig. 9: ein weiteres Ausführungsbeispiel einer erfindungsgemäßen Vorrichtung
- Fig. 10: ein Diagramm zur Visualisierung eines Sterilisationszyklusses, und
- Fig. 11A bis 11D: weitere Detailansichten zur Beschreibung der Funktionsweise der erfindungsgemäßen Vorrichtung.

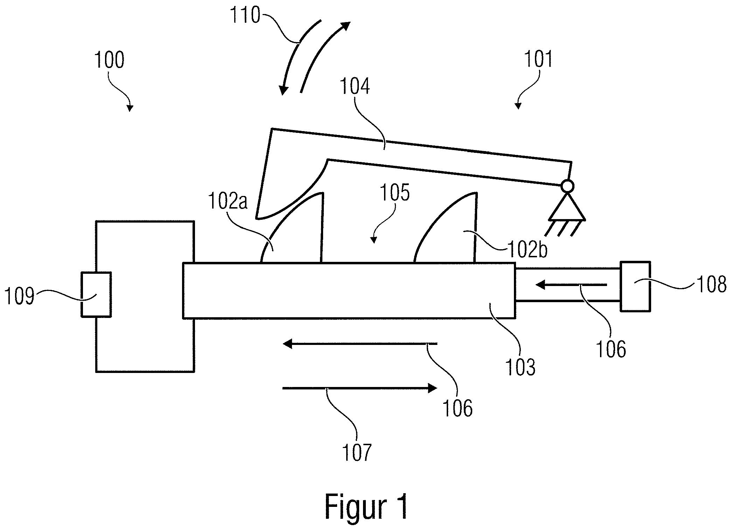

Figur 1 zeigt ein schematisches Bild einer erfindungsgemäßen Vorrichtung 100. Die Vorrichtung 100 weist einen Rastmechanismus 101 auf. Der Rastmechanismus 101 weist ein Rastenelement 103 und eine Klinke 104 auf. Das Rastenelement 103 weist mindestens zwei Rasten 102a, 102b auf. Zwischen den beiden Rasten 102a, 102b ist ein Rastenzwischenraum 105 ausgebildet, in den die Klinke 104 eingreifen kann.

Das Rastenelement 103 ist in einer Freilaufrichtung 106 relativ zu der Klinke 104 bewegbar. In einer Sperrrichtung 107 hingegen ist eine Bewegung des Rastenelements 103 relativ zu der Klinke 104 mittels der Klinke 104 blockierbar. Dies kann beispielsweise durch eine, wie in Figur 1 gezeigte, geeignete Form der Klinke 104 und der einzelnen Rasten 102a, 102b realisiert werden.

Die Raste 102a weist eine abgerundete Seite und eine flache Seite auf. Die Klinke 104 weist ebenfalls eine abgerundete und eine flache Seite auf. Während in Freilaufrichtung 106 die beiden abgerundeten Seiten übereinander hinweg gleiten, blockieren die jeweils flachen Seiten eine Bewegung der Klinke 104 relativ zu der Raste 102a in Sperrrichtung 107.

Unter einer Relativbewegung des Rastenelements 103 und der Klinke 104 relativ zueinander ist eine Bewegung der beiden Elemente 103, 104 relativ zueinander in Freilaufrichtung 106 gemeint. Das heißt, eine Verschiebung der beiden Elemente 103, 104 relativ zueinander. Die Bewegung der Klinke 104 beim Eingreifen in den Rastenzwischenraum 105 sowie die Bewegung beim Lösen des Eingriffs ist in der Regel eine Auf- und Abbewegung 110, die quer zur Freilaufrichtung 106 gerichtet ist. Diese Auf- und Abbewegung 110 der Klinke 104 ist im Sinne der vorliegenden Offenbarung jedoch nicht unter der in der Beschreibung vorkommenden Relativbewegung zwischen dem Rastenelement 103 und der Klinke 104 gemeint.

Die erfindungsgemäße Vorrichtung 100 weist ferner ein auslenkbares Betätigungsmittel 108 auf. Das Betätigungsmittel 108 ist ausgebildet, um mittels einer Auslenkung das Rastenelement 103 und die Klinke 104 relativ zueinander rastenweise in Freilaufrichtung 106 zu bewegen.

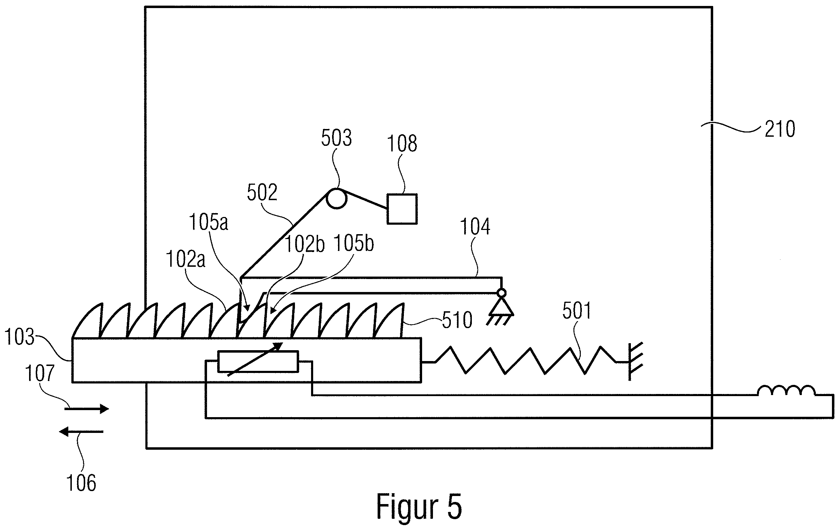

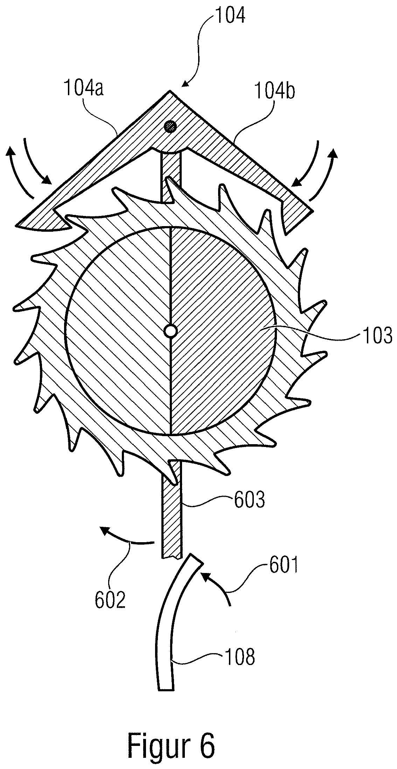

In dem in Figur 1 abgebildeten Ausführungsbeispiel ist das Betätigungsmittel 108 ein Linearaktuator, der linear entlang bzw. parallel zu der Freilaufrichtung 106 auslenkbar ist. Mittels dieser Auslenkbewegung betätigt das Betätigungsmittel 108 in diesem Falle das Rastenelement 103 und schiebt es relativ zu der Klinke 104 rastenweise weiter. Es ist aber ebenso denkbar, dass das Betätigungsmittel 108 die Klinke 104 mittels einer geeigneten Auslenkbewegung betätigt. Ein solches Ausführungsbeispiel wird nachfolgend unter Bezugnahme auf die Figuren 5 und 6 näher erläutert.

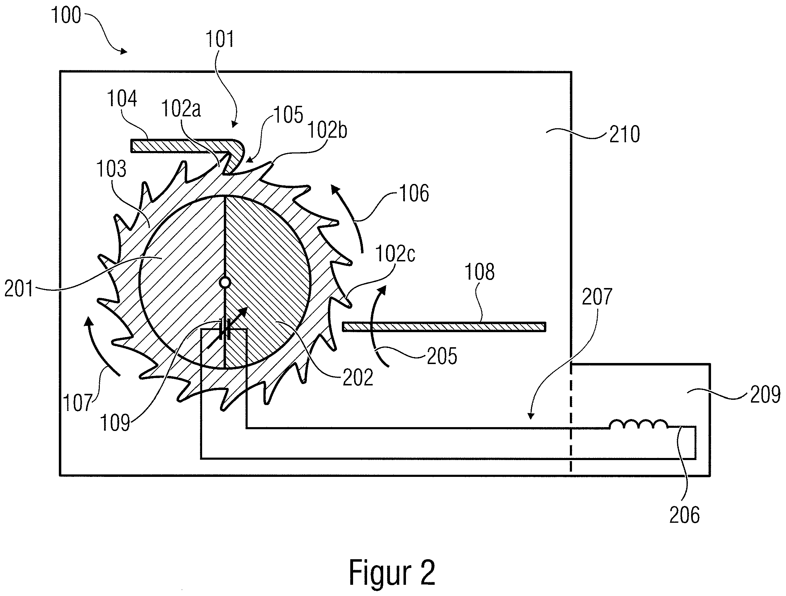

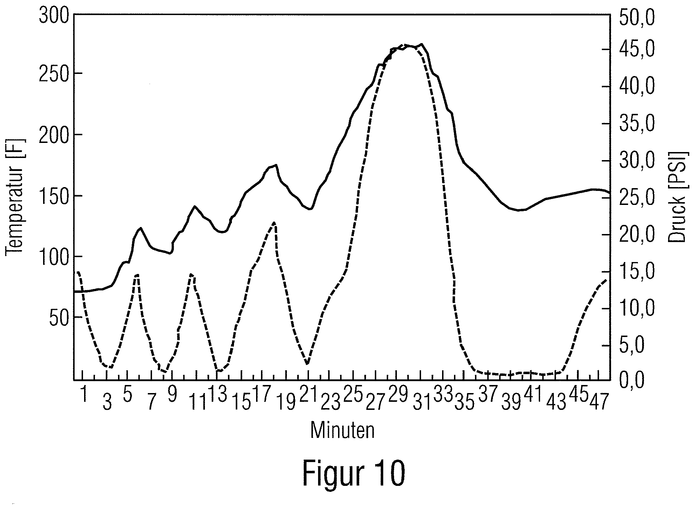

Generell gilt für alle Ausführungsformen, dass das auslenkbare Betätigungsmittel 108 auch beispielsweise mittels einer Schwenkbewegung die Klinke 104 bzw. das Rastenelement 103 bewegen bzw. betätigen kann. Beispielsweise wäre es denkbar, dass das Betätigungsmittel 108 derart ausgebildet ist, dass es sich verformt (z.B. durch Zuführen externer Energie, wie beispielsweise thermischer und/oder elektrischer und/oder mechanischer Energie) und während des Verformungsvorgangs die Klinke 104 oder das Rastenelement 103 bewegt bzw. betätigt. Derartige Ausführungsformen werden nachfolgend ausführlich mit Bezug auf die Figuren 2, 3A bis 3F, 6, 7A bis 7C, 9A bis 9D, 10 und 11 näher beschrieben.

Die erfindungsgemäße Vorrichtung 100 weist außerdem ein elektrisches Bauelement 109 auf. Das elektrische Bauelement 109 ist ausgebildet, um seine elektrische Eigenschaft in Abhängigkeit von der rastenweisen Bewegung des Rastenelements 103 relativ zu der Klinke 104 zu verändern. In dem in Figur 1 abgebildeten Ausführungsbeispiel verändert das elektrische Bauelement 109 seine elektrische Eigenschaft jedes Mal, wenn das Betätigungsmittel 108 das Rastenelement 103 um eine Raste 102a, 102b weiter schiebt.

Bei dem elektrischen Bauelement 109 kann es sich beispielsweise um einen variablen ohm'schen Widerstand handeln, der seinen Widerstandswert in Abhängigkeit von der Position des Rastenelements 103 relativ zu der Klinke 104 verändert. Das elektrische Bauelement 109 kann aber auch ein Kondensator sein, der seine Kapazität in Abhängigkeit von der Position des Rastenelements 103 relativ zu der Klinke 104 verändert. Ebenso denkbar wäre es, dass das elektrische Bauelement 109 eine Spule wäre, die ihre Induktivität in Abhängigkeit von der Position des Rastenelements 103 relativ zu der Klinke 104 verändert.

Figur 2 zeigt ein weiteres Ausführungsbeispiel einer erfindungsgemäßen Vorrichtung 100. Auch hier weist der Rastmechanismus 101 eine Klinke 104 und ein Rastenelement 103 mit mindestens zwei Rasten 102a, 102b auf, wobei die Klinke 104 in einen Rastenzwischenraum 105 zwischen den beiden Rasten 102a, 102b eingreifen kann.

Das Rastenelement 103 ist hier als ein Zahnrad ausgebildet. Die einzelnen Rasten 102a, 102b sind in Form einer radial außenseitig an dem Zahnrad 103 angeordneten Verzahnung ausgebildet. Es wäre aber auch denkbar, dass die einzelnen Zähne 102a, 102b in Form einer radial innenseitig angeordneten Verzahnung an dem Zahnrad 103 ausgebildet sind.

Das Zahnrad 103 ist hier als ein frei drehbares Zahnrad ausgebildet. Es wäre aber ebenfalls denkbar, dass das Zahnrad 103 elastisch drehbar wäre, d.h. das Zahnrad 103 könnte beispielsweise entgegen einer Federkraft drehbar sein. Eine Feder (hier nicht abgebildet), wie z.B. eine aus Uhrwerken bekannte Spiralfeder, könnte mit dem Zahnrad 103 gekoppelt sein, sodass sich beim Bewegen des Zahnrads 103 in eine erste Richtung die Feder spannt (d.h. entweder auf Druck oder auf Zug spannt), und beim Bewegen des Zahnrads 103 in eine der ersten Richtung entgegengesetzte zweite Richtung die Feder entspannt.

Bei dem in Figur 2 gezeigten frei drehbaren Zahnrad 103 greift die Klinke 104 in die Zahnzwischenräume 105 zwischen zwei Zähnen 102a, 102b des Zahnrads 103 ein. Es ist zu erkennen, dass sich auch hier, aufgrund der spezifischen Form der Klinke 104 und der einzelnen Zähne 102a, 102b des Zahnrads 103, eine Freilaufrichtung 106 ergibt, in der das Zahnrad 103 relativ zu der Klinke 104 frei drehbar ist. In der entgegengesetzten Richtung, d.h. in einer Sperrrichtung 107, blockiert die Klinke 104 jedoch die Bewegung des Zahnrads 103.

In diesem Ausführungsbeispiel betätigt das Betätigungsmittel 108 das Rastenelement 103, um das Rastenelement 103 relativ zu der Klinke 104 in Freilaufrichtung 106 rastenweise um jeweils eine Raste 102a, 102b weiterzubewegen. Wie zu erkennen ist, greift das Betätigungsmittel 108 dabei an einer Raste 102c des Rastenelements 103 an, um das Rastenelement 103 relativ zu der Klinke 104 rastenweise weiterzubewegen.

Die erfindungsgemäße Vorrichtung 100 ist auf einem Substrat 210 bereitgestellt. Das Substrat 210 kann beispielsweise ein Siliziumwafer sein. Die erfindungsgemäße Vorrichtung 100 ist außerdem auf dem Substrat 210 als ein MEMS (microelectromechanical) System bereitgestellt. Beispielsweise kann die abgebildete Zahnradstruktur 103 durch geeignete Ätzverfahren hergestellt werden.

Wie durch den Pfeil 205 angedeutet ist, wird das Betätigungsmittel 108 in der Bildebene nach oben ausgelenkt, um das Zahnrad 103 zu betätigen. Die Auslenkung des Betätigungsmittels 108 geschieht also in der Substratebene, d.h. in einer Ebene parallel bzw. horizontal zu der Substratebene.

Bei der Bewegung des Betätigungsmittels 108 handelt es sich im Wesentlichen um eine durch Zuführen externer Energie (z.B. thermischer Energie) bedingte Schwenkbewegung, wobei das Verhalten des Betätigungsmittels 108 etwa mit dem eines einseitig eingespannten Biegebalkens vergleichbar ist.

Das elektrische Bauelement 109 ist hier als ein Kondensator ausgebildet. Genauer gesagt ist hier eine erste Kondensatorplatte 201 an dem Substrat 210 bereitgestellt und eine zweite Kondensatorplatte 202 ist an dem Rastenelement 103 bereitgestellt.

Wie zu erkennen ist, handelt es sich bei den beiden Kondensatorplatten 201, 202 um zwei halbkreisförmige Segmente. In der in Figur 2 abgebildeten Stellung des Zahnrads 103 relativ zu dem Substrat 210 sind die beiden Kondensatorplatten 201, 202 derart zueinander ausgerichtet, dass sie sich genau gegenüberliegen, d.h. sodass sie sich zu einem in Draufsicht ganzen Kreis zusammenfügen.

Bedingt durch die Stellung der beiden Kondensatorplatten 201, 202 relativ zueinander weist der Kondensator 109 in dieser Stellung eine bestimmte Kondensatorkapazität auf. Bei einer rastenweisen Bewegung des Rastenelements 103 relativ zu dem Substrat 210 dreht sich das Zahnrad 103 relativ zu dem Substrat 210 und somit verändert sich auch die Ausrichtung der beiden Kondensatorplatten 201, 202 relativ zueinander. Gleichzeitig verändert sich damit auch die Kondensatorkapazität des Kondensators 109.

In dem in Figur 2 abgebildeten Ausführungsbeispiel ist das elektrische Bauelement, d.h. der Kondensator 109, ein verstellbares Glied eines RFID-Schwingkreises 207. Der RFID-Schwingkreis 207 weist neben dem Kondensator 109 auch noch eine Spule 206 auf. Hierbei handelt es sich also um einen LC-Schwingkreis mit bauteilabhängiger Resonanzfrequenz.

Die Resonanzfrequenz des Schwingkreises 207 ändert sich in Abhängigkeit der Kondensatorkapazität des verstellbaren Kondensators 109. So ergibt sich für jede Position des Zahnrads 103 relativ zu dem Substrat 210 bzw. relativ zu der Klinke 104 eine bestimmte Stellung der beiden Kondensatorplatten 201, 202 zueinander. Dadurch bedingt stellt sich für jede Position eine bestimmte Kondensatorkapazität und somit eine bestimmte Resonanzfrequenz des RFID-Schwingkreises 207 ein.

Das heißt der RFID-Schwingkreis 207 weist für jede Position des Zahnrads 103 relativ zu dem Substrat 210 bzw. relativ zu der Klinke 104 eine spezifische Resonanzfrequenz auf. Der RFID-Schwingkreis 207 kann mittels eines geeigneten RFID-Lesegeräts ausgelesen werden. Hierbei kann also von der jeweils charakteristischen Übertragungs-Frequenz des Schwingkreises 207 auf die Position des Zahnrads 103 (zweite Kondensatorplatte 202) relativ zu dem Substrat 210 (erste Kondensatorplatte 201) bzw. relativ zu der Klinke 104 rückgeschlossen werden.

Die Vorrichtung 100 kann hierfür eine elektronische Schnittstelle 209 aufweisen. Die elektronische Schnittstelle 209 muss nicht notwendigerweise Teil des MEMS-Elements selbst sein. Die elektronische Schnittstelle 209 ermöglicht ein Auslesen der Veränderung des elektrischen Bauelements 109, z.B. wenn das elektrische Bauelement 109 direkt das verstellbare Glied eines RFID Schwingkreises oder einer aufwendigeren Elektronik darstellt.

Entspricht also das elektrische Bauelement 109 einem typischen Element (Kondensator, Spule, Widerstand) eines Schwingkreises 207, z.B. einem veränderlichen Kondensator 109, und bildet dieser zusammen mit einer Spulenstruktur 206 einen LC-Schwingkreis 207, so ändert eine Veränderung der Kapazität auch die Schwingcharakteristik des Schwingkreises 207. Der so entstehende passive Transponder eines RFID-Systems (engl. radio-frequency identification) kann mit einem entsprechenden Lesegerät drahtlos ausgelesen werden.

Ist das elektrische Bauelement 109 Teil eines elektronischen Schaltkreises, der wiederum Teil eines RFID-Transpondersystems ist, so kann mit Hilfe eines entsprechenden Lesegeräts elektrische Energie drahtlos in den Schaltkreis eingekoppelt werden und zum Ausführen von Funktionen des elektronischen Schaltkreises verwendet werden, z.B. zu Signalverstärkungen, -auswertungen, und weiteren Sendeaufgaben.

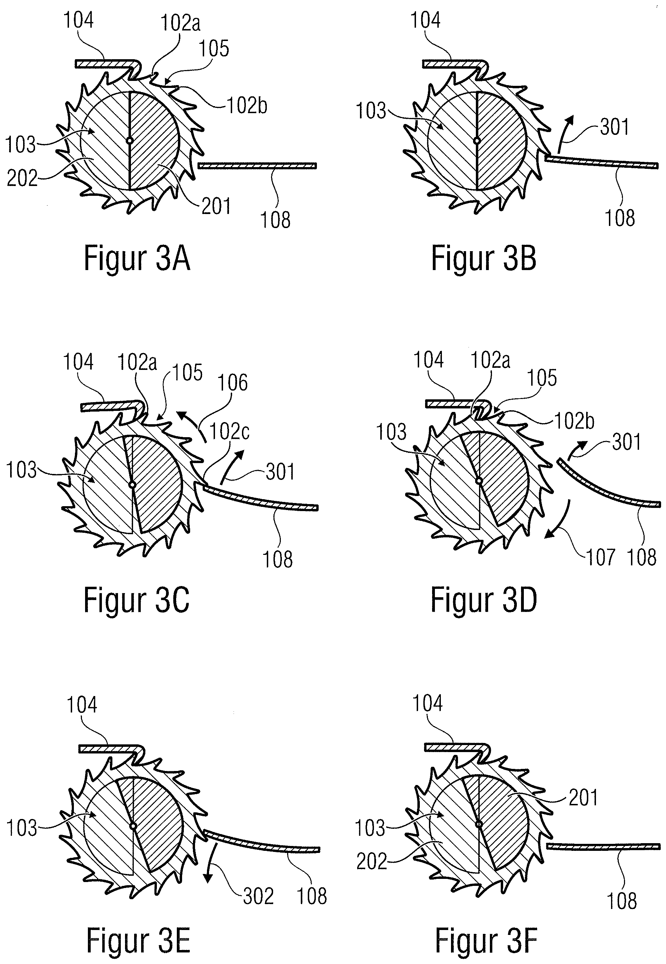

Die Figuren 3A bis 3F zeigen Detailansichten einer Auslenkung des Betätigungsmittels 108 und einer damit verbundenen Betätigung des in Figur 2 dargestellten Zahnrads 103 relativ zu dem Substrat 210 bzw. relativ zu der Klinke 104.

In Figur 3A sind der Rastmechanismus 101, aufweisend die Klinke 104 und das Zahnrad 103, sowie das Betätigungsmittel 108 in einer Ausgangslage gezeigt. Alle Elemente 103, 104, 108 stehen weitestgehend still.

In Figur 3B ist gezeigt, wie das Betätigungsmittel 108 beginnt sich zu bewegen. Das Betätigungsmittel 108 wird in der Bildebene nach oben ausgelenkt, d.h. entlang der mit dem Pfeil 301 symbolisierten Richtung. Dabei berührt das Betätigungsmittel 108 abschnittsweise einen der Zähne des Zahnrads 103 und kommt mit diesem zur Anlage.

In Figur 3C ist zu sehen, wie das Betätigungsmittel 108 weiter in Pfeilrichtung 301 nach oben ausgelenkt wird. Dabei nimmt das Betätigungsmittel 108 das Zahnrad 103 mit, indem es auf den mit dem Betätigungsmittel 108 in Eingriff stehenden Zahn 102c des Zahnrads 103 wirkt. Das Zahnrad 103 beginnt sich in Freilaufrichtung 106 zu drehen und die Klinke 104 rutscht entlang der Zahnflanke des Zahns 102, sodass die Klinke 104 nach oben angehoben wird.

In Figur 3D ist zu sehen, dass sich das Betätigungsmittel 108 weiter nach oben, d.h. in Pfeilrichtung 301 bewegt, um das Zahnrad 103 somit weiter zu drehen. Bei weiterer Drehung des Zahnrads 103 rutscht die Klinke 104 über den Zahn 102a hinweg und greift in den nächsten Zahnzwischenraum 105 zwischen den Zähnen 102a und102b ein. Die Klinke 104 blockiert nun eine rückwärtsgerichtete Bewegung des Zahnrads 103 in Sperrrichtung 107.

Wie in Figur 3E zu erkennen ist, bewegt sich das Betätigungsmittel 108 nun wieder in seine Ausgangsposition zurück. Dabei wird das Betätigungsmittel 108 wieder in die entgegengesetzte Richtung, dargestellt durch den Pfeil 302, d.h. in der Bildebene nach unten, ausgelenkt. Das Betätigungsmittel 108 gleitet dabei über die Zähne des Zahnrads 103 hinweg, da das Zahnrad 103 mittels der Klinke 104 gegen ein Verdrehen in Sperrrichtung 107 gesichert ist.

In Figur 3F ist zu erkennen, dass das Betätigungsmittel 108 wieder in seine Ausgangslage zurückgekehrt ist. Das Betätigungsmittel 108 kann nun bei seiner nächsten Auslenkung in Pfeilrichtung 301 in den nächsten Zahn des Zahnrads 103 eingreifen und das Zahnrad 103 in der oben beschriebenen Art und Weise wieder um einen Zahn weiterdrehen. Der beschriebene Mechanismus aus Betätigungsmittel 108, Rastenelement 103 und Klinke 104 ist erfindungsgemäß derart ausgebildet, dass das Rastenelement 103 relativ zu der Klinke 104 nur rastenweise, d.h. immer nur um eine Raste, weiterbewegt wird.

In Figur 3F ist außerdem zu erkennen, wie sich die erste, an dem Zahnrad 103 angeordnete, Kondensatorplatte 201 relativ zu der zweiten, in dem Substrat 210 vorgesehenen, Kondensatorplatte 202 verschiebt. Dadurch ändert sich die Kondensatorkapazität im Vergleich zu der in Figur 3A gezeigten Ausgangslage.

In dem in den Figuren 3A bis 3F dargestellten Ausführungsbeispiel sind die beiden Kondensatorplatten 201, 202 derart zueinander angeordnet, dass sie sich in der in Figur 3A gezeigten Ausgangslage nicht überschneiden bzw. überlappen. Die Kondensatorkapazität weist hier im Vergleich zu allen anderen einnehmbaren Positionen des Zahnrads 103 die geringste Kapazität auf, bzw. ist die Kapazität hier etwa Null. Mit zunehmender Rotation des Zahnrads 103 überlappen sich die beiden Kondensatorplatten 201, 202 immer mehr, sodass die Kondensatorkapazität immer weiter steigt. Bei einer Umdrehung von 180° überlappen sich die beiden Kondensatorplatten 201, 202 vollständig, sodass der Kondensator 109 in dieser Stellung im Vergleich zu allen anderen einnehmbaren Positionen des Zahnrads 103 die größte Kapazität aufweist. Mit fortschreitender Rotation des Zahnrads 103 nimmt die Kondensatorkapazität wieder ab.

Die Kondensatorplatten 201, 202 sind hier als halbkreisförmige Platten ausgebildet, wobei auch andere geeignete Formen denkbar wären.

Bei der Bewegung des Betätigungsmittels 108 handelt es sich im Wesentlichen um eine durch Zuführen externer Energie (z.B. thermischer Energie) bedingte Schwenkbewegung, wobei das Verhalten des Betätigungsmittels 108 etwa mit dem eines einseitig eingespannten Biegebalkens vergleichbar ist.

Das elektrische Bauelement 109 muss nicht zwingend ein Kondensator sein. Es wäre ebenso denkbar, dass das elektrische Bauelement 109 ein ohm'scher Widerstand, eine Spule oder ein elektrooptisches Element ist.

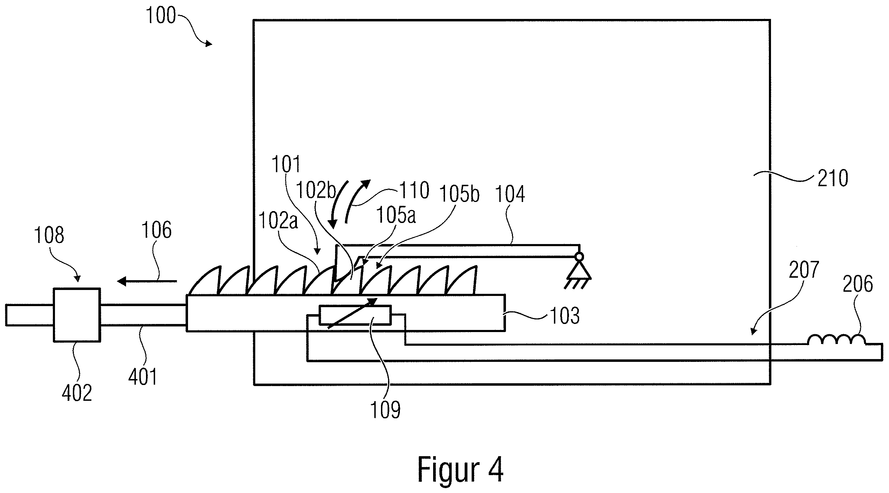

Ein Ausführungsbeispiel, bei dem sich beispielsweise ein ohm'scher Widerstand anbieten könnte, ist in Figur 4 gezeigt. Der Aufbau der hier abgebildeten erfindungsgemäßen Vorrichtung 100 ist ähnlich zu dem Beispiel aus Figur 1. Auch hier ist ein Rastmechanismus 101 auf einem Substrat 210 angeordnet. Außerdem ist das Betätigungsmittel 108 ebenfalls als ein Linearaktuator ausgebildet, der das Rastenelement 103 betätigt.

Das Rastenelement 103 ist hier als eine mehrere Zähne aufweisende Zahnstange ausgebildet. Im Gegensatz zu dem in Figur 1 gezeigten Beispiel drückt jedoch das Betätigungselement 103 nicht die Zahnstange 103 in Freilaufrichtung 106, sondern die Zahnstange 103 wird von dem Betätigungselement 108 in Freilaufrichtung 106 gezogen.

Bei jedem rastenweisen Weiterziehen der Zahnstange 103 mittels des Betätigungsmittels 108 bewegt sich die Klinke 104 entlang der durch die Pfeile 110 gezeigten Richtungen auf und ab, um so zwischen den einzelnen Zähnen 102a, 102b in den jeweiligen Zahnzwischenraum 105a einzugreifen.

Das Rastenelement 103 ist hier zwar als eine lineare Zahnstange dargestellt. Es ist aber ebenfalls denkbar, dass die Zahnstange 103 nicht linear sondern gekrümmt ist. So kann eine Zahnstange 103 beispielsweise auch eine Kreisbogen- bzw. Kreissegment-förmige Struktur aufweisen, wobei die Verzahnung radial innenseitig und/oder radial außenseitig angeordnet sein kann.

Unabhängig davon, ob es sich bei dem Rastenelement 103 nun um eine Zahnstange oder, wie in Figur 2 dargestellt, um ein frei bewegliches Zahnrad handelt, sind erfindungsgemäß die einzelnen Rasten 102a, 102b entlang des Rastenelements 103 in Freilaufrichtung 106 hintereinander angeordnet, sodass die Klinke 104 bei der rastenweisen Bewegung nacheinander von einem Rastenzwischenraum 105a in den jeweils nächsten benachbarten Rastenzwischenraum 105b eingreift. Dadurch muss beispielsweise bei dem sich endlos drehenden Zahnrad 103 kein, wie im Stand der Technik benötigter, separater Rückstellmechanismus bereitgestellt werden.

Das Betätigungsmittel 108 kann beispielsweise ein Zugmittel 401 sowie eine Zugvorrichtung 402 aufweisen, die mittels des Zugmittels 401 das Betätigungselement 103 betätigt. Gemäß Ausführungsbeispielen der Erfindung kann das Betätigungsmittel 108 beispielsweise mechanisch (hierzu zählt auch thermisch) oder elektrisch auslenkbar sein.

Das elektrische Bauelement 109 kann, wie eingangs erwähnt, ein variabler ohm'scher Widerstand sein. In dem hier gezeigten Ausführungsbeispiel ist der Widerstand 109 zwischen dem Rastmechanismus 101 und dem Substrat 210 angeordnet. Der variable ohm'sche Widerstand 109 ist etwa vergleichbar mit einem Potentiometer. Mit jeder rastenweisen Weiterbewegung des Rastenelements 103 relativ zu der Klinke 104 bzw. relativ zu dem Substrat 210 verändert sich dessen elektrischen Widerstand.

Der ohm'sche Widerstand 109 kann ebenfalls Bestandteil eines Schwingkreises 207 sein. Hier handelt es sich beispielsweise um einen verstimmbaren RL-Schwingkreis 207, der den erwähnten ohm'schen Widerstand 109 sowie ein Spulenanordnung 206 aufweist.

Auch in diesem Ausführungsbeispiel kann statt des ohm'schen Widerstandes auch ein Kondensator als verstimmbares elektrisches Bauelement 109 zum Einsatz kommen, um zusammen mit der Spule 206 einen, wie zuvor mit Bezug auf Figur 2 beschriebenen, verstimmbaren LC-Schwingkreis 207 zu bilden.

Wie ebenfalls zuvor mit Bezug auf Figur 2 erwähnt, kann das Rastenelement 103 frei beweglich, oder aber auch elastisch beweglich sein. Während Figur 4 eine frei bewegliche Zahnstange 103 gezeigt hat, ist in Figur 5 eine mit einer Feder 501 vorgespante Zahnstange 103 gezeigt, die somit elastisch beweglich ist.

Figur 5 zeigt also ein weiteres Ausführungsbeispiel der erfindungsgemäßen Vorrichtung 100. Die hier abgebildete Vorrichtung 100 entspricht im Wesentlichen der zuvor mit Bezug auf Figur 4 beschriebenen Ausführungsform. Ein Unterschied besteht jedoch darin, dass das Betätigungsmittel 108 hier nicht das Rastenelement 103 sondern die Klinke 104 betätigt.

Das Rastenelement 103 ist hier mittels eines Spannelements 501 vorgespannt. Das Spannelement 501 kann beispielsweise eine Zugfeder sein, die im Ausgangszustand auseinandergezogen und somit vorgespannt ist. In dem hier abgebildeten Beispiel könnte das Rastenelement 103 bis zu dem letzten Zahn 510 in Freilaufrichtung 106 (in Figur 5 der Zahn rechts außen) entgegen der Zugkraft der Zugfeder 501 aufgezogen werden. Die Klinke 104 greift in den letzten Zahnzwischenraum ein und sperrt die Bewegung des Rastenelements 103 in Sperrrichtung 107.

Wie eingangs erwähnt, betätigt das Betätigungselement 108 die Klinke 104. Das Betätigungselement 108 muss hierbei nicht direkt die Klinke 104 kontaktieren, sondern das Betätigungselement 108 kann beispielsweise auch mittels eines Verbindungsmittels 502 mit der Klinke 104 verbunden sein. Das Betätigungselement 108 kann außerdem optional eine Umlenkvorrichtung 503 aufweisen, sodass das Betätigungsmittel 108 die Klinke 104 nicht zwangsläufig in dieselbe Richtung wie die Auslenkrichtung des Betätigungsmittels 108 bewegen muss. Das eben gesagte gilt natürlich ebenso für ein Betätigungsmittel 108, das das Rastenelement 103 betätigt.

Wie eingangs erwähnt, ist also das Rastenelement 103 mittels der Zugfeder 501 vorgespannt, d.h. die Zugfeder 501 zieht das Rastenelement 103 in Sperrrichtung 107. Die Klinke 104 sperrt jedoch die Bewegung des Rastenelements 103 in ebendiese Sperrrichtung 107. Bei einer den Eingriff in einen Rastenzwischenraum 105a lösenden Bewegung der Klinke 104 bewegt sich das vorgespannte Rastenelement 103 aufgrund der Vorspannung des Spannelements 501, d.h. die Zugfeder 501 zieht das Rastenelement 103 nun in Sperrrichtung 107. Dies ist nur möglich, da sich ja die Klinke 104 aus dem Eingriff mit dem Rastenzwischenraum 105a gelöst hat. Erfindungsgemäß schiebt sich das Rastenelement 103 aber jeweils immer nur um eine Raste 102a, 102b weiter, bevor die Klinke 104 wieder in einen benachbarten nächsten Rastenzwischenraum 105b eingreift.

Anstatt der eben beschriebenen Zugfeder könnte auch eine Druckfeder vorgesehen sein, die das Rastenelement 103 in Sperrrichtung 107 drückt. In diesem Fall würde die Druckfeder jedoch im Vergleich zu Figur 5 auf der gegenüberliegenden Seite des Rastenelements 103 angreifen.

Um ein ungebremstes Durchrutschen des Rastenelements 103 zu der (kurzzeitig nicht eingreifenden) Klinke 104 zu verhindern, kann eine Bremsvorrichtung, wie eine weitere Klinke, oder ein mechanischer Anschlag vorgesehen sein.

Figur 6 zeigt einen Ausschnitt einer solchen Ausführungsform, bei der das Betätigungsmittel 108 die Klinke 104 betätigt. Dieses Prinzip ähnelt dem bekannten Prinzip der Ankerhemmung, wie es beispielsweise in Uhrwerken eingesetzt wird. Das als Zahnrad ausgebildete Rastenelement 103 ist vorgespannt, z.B. mittels einer geeigneten Feder, z.B. mittels einer aus Uhrwerken bekannten Spiralfeder (hier nicht abgebildet).

Die Klinke 104 ist hier als eine Doppelklinke ausgebildet, d.h. sie weist einen ersten bzw. linken Klinkenteil 104a und einen zweiten bzw. rechten Klinkenteil 104b auf. Die Klinke 104 ist an einem Stab 103 befestigt. Auf der der Klinke 104 gegenüberliegenden Seite des Stabs 603 greift das Betätigungsmittel 108 an.

Eine Auslenkung des Betätigungsmittels 108 in Pfeilrichtung 601 schwenkt den Stab 603 in Pfeilrichtung 602 aus. Durch diese Schwenkbewegung wird der Eingriff des ersten Klinkenteils 104a in den Zahnzwischenraum des Zahnrads 103 gelöst und der zweite Klinkenteil 104b kommt in Eingriff mit einem nächsten benachbarten Zahnzwischenraum. Zwischen den aus- und eingreifenden Bewegungen der beiden Klinkenteile 104a, 104 springt das Zahnrad 103 aufgrund seiner Vorspannung um einen Zahn weiter.

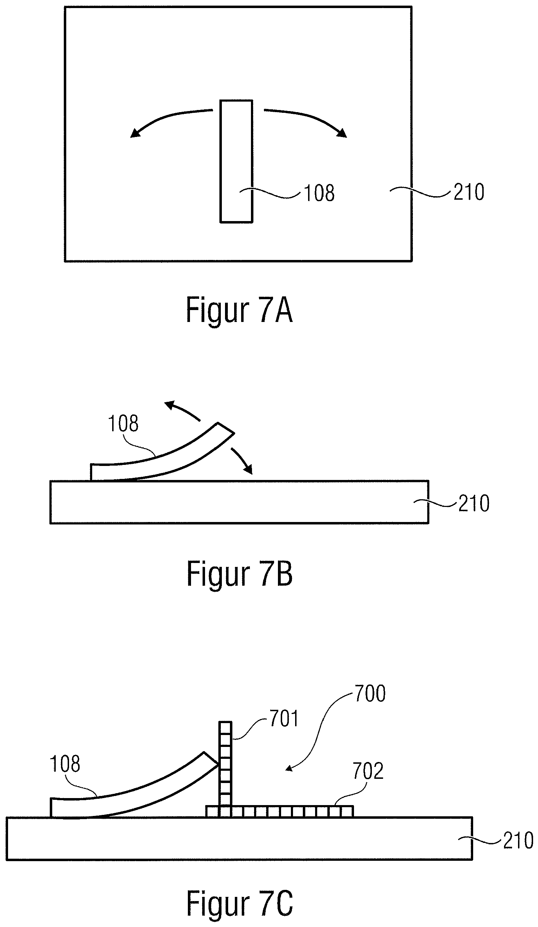

In all den vorgenannten Ausführungsbeispielen kann die Bewegung des Betätigungsmittels 108 in der bzw. parallel zu der Substratebene geschehen. Figur 7A zeigt eine Draufsicht auf ein Substrat 210 und einem an dem Substrat 210 bereitgestellten Betätigungsmittel 108. Wie mit den Pfeilen angedeutet, kann das Betätigungsmittel 108 beispielsweise in die eingezeichneten Richtungen ausschwenken. Dies entspricht einer Bewegung in der bzw. parallel zu der Substratebene.

Figur 7B zeigt eine Seitenansicht eines Substrats 210 und eines Betätigungsmittels 108. Hier bewegt sich das Betätigungsmittel 108 in einer Ebene senkrecht zu der Substratebene.

Figur 7C zeigt ein weiteres Ausführungsbeispiel, wobei eine Umlenkvorrichtung 700 vorgesehen ist, die ausgebildet ist, um die Bewegung des Betätigungsmittels 108 in einer Ebene senkrecht zu der Substratebene in eine Bewegung umzulenken, die wiederum in einer Ebene horizontal bzw. parallel zu der Substratebene verläuft.

Hierfür kann die Umlenkvorrichtung 700 beispielsweise ein erstes, vertikal zur Substratebene angeordnetes Zahnrad 701 und ein zweites, parallel bzw. horizontal zur Substratebene angeordnetes Zahnrad 702 aufweisen, wobei die beiden Zahnräder 701, 702 ineinandergreifen. Das Betätigungsmittel 108 wird ausgelenkt und betätigt dabei das erste Zahnrad 701. Das erste Zahnrad 701 dreht sich in einer Ebene senkrecht zur Substratebene und betätigt dabei wiederum das zweite Zahnrad 702. Die beiden Zahnräder 701, 702 können beispielsweise mit einer Schnecke-Kegelrad-Verzahnung miteinander in Eingriff gelangen.

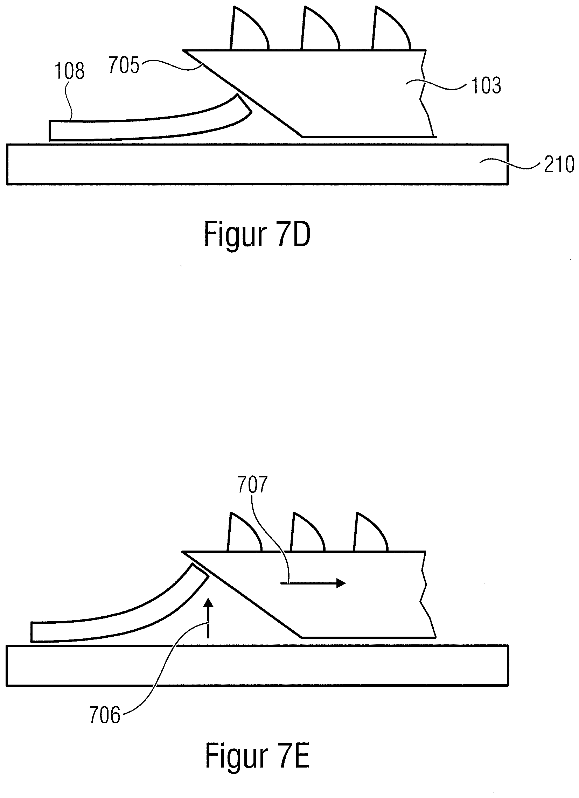

Figur 7D zeigt eine weitere Möglichkeit zur mikrosystemtechnischen Umsetzung einer Umlenkbewegung. In Figur 7D ist ein Substrat 210, ein Betätigungsmittel 108 und ein Rastenelement 103 gezeigt. Das Rastenelement 103 weist eine dem Betätigungsmittel 108 zugewandte schräge Fläche 705 auf.

In Figur 7E ist gezeigt, wie eine nach oben, d.h. senkrecht zur Substratebene gerichtete Bewegung 706 des Betätigungsmittels 108 in eine parallel zur Substratebene gerichtete Bewegung 107 des Rastenelements 103 erfolgen kann. Dabei gleitet zumindest ein Abschnitt des Betätigungsmittels 108 während des Auslenkvorgangs an der schrägen Fläche 705 des Rastenelements 103 entlang und schiebt dieses somit in die in Pfeilrichtung 107 dargestellte Richtung parallel zur Substratebene.

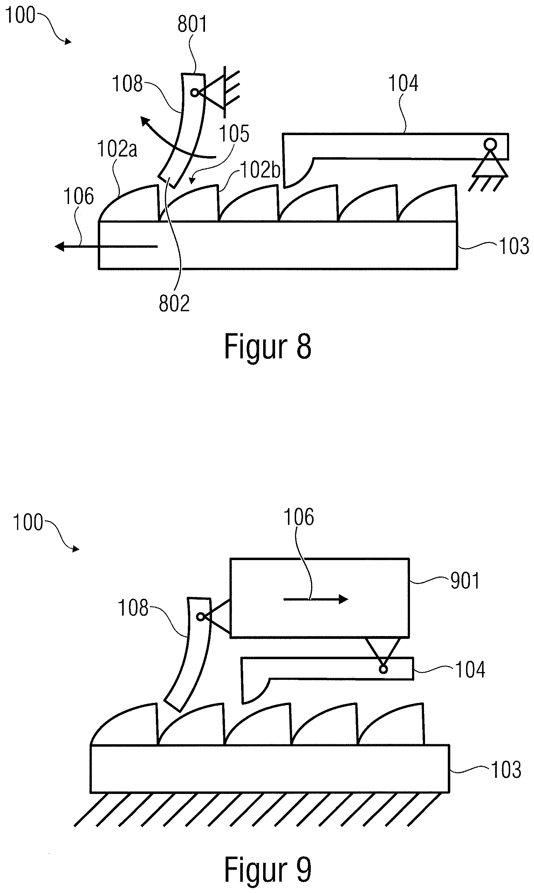

Figur 8 zeigt ein weiteres Ausführungsbeispiel einer erfindungsgemäßen Vorrichtung 100. Der Unterschied zu den zuvor beschriebenen Ausführungsformen besteht unter anderem darin, dass das Betätigungsmittel 108 in einen Rastenzwischenraum 105 zwischen zwei benachbarten Rasten 102a, 102b eingreift, um so das als Zahnstange ausgebildete Rastenelement 103 rastenweise weiterzubewegen.

Im Endeffekt entspricht diese Vorrichtung der zuvor mit Bezug auf Figur 2 beschriebenen Vorrichtung, jedoch mit dem Unterschied, dass es sich bei dem Rastenelement 103 nicht um ein frei drehbares Zahnrad sondern um eine Zahnstange handelt. Auch die Zahnstange 103 kann frei beweglich oder elastisch beweglich, d.h. mit einer Zug- oder Druckfeder vorgespannt, sein.

In dem in Figur 8 abgebildeten Ausführungsbeispiel ist es vorteilhaft, wenn das Betätigungsmittel 108 etwa senkrecht zu der Erstreckungsrichtung der Zahnstange 103 ausgebildet ist. Somit kann, wie gezeigt, ein Biegewandler als auslenkbares Betätigungsmittel 108 eingesetzt werden, wobei das dem eingespannten Ende 801 gegenüberliegende freie Ende 802 des Biegewandlers 108 in den Zahnzwischenraum 105 eingreift.

Figur 9 zeigt ein weiteres Ausführungsbeispiel einer erfindungsgemäßen Vorrichtung 100. Der Unterschied zu den zuvor beschriebenen Ausführungsbeispielen besteht unter anderem darin, dass das Rastenelement 103 fixiert ist und sowohl die Klinke 104 als auch das auslenkbare Betätigungsmittel 108 gemeinsam an einer gegenüber dem fixierten Rastenelement 103 beweglichen Vorrichtung 901 angeordnet sind. Bei der beweglichen Vorrichtung 901 kann es sich beispielsweise um eine Art Schlitten handeln.

Das Betätigungsmittel 108 ist an der beweglichen Vorrichtung 901 angeordnet und einseitig an der beweglichen Vorrichtung 901 eingespannt. Das freie Ende des Betätigungsmittels 108 greift in einen Zahnzwischenraum zwischen zwei benachbarten Zähnen ein.

An der beweglichen Vorrichtung 901 ist außerdem die Klinke 104 angeordnet. Die Klinke 104 ist schwenkbar an der beweglichen Vorrichtung 901 angeordnet, sodass die Klinke 104 in einen Zahnzwischenraum eingreifen und aus einem Eingriff mit einem Zahnzwischenraum auch wieder ausschwenken kann.

Eine Auslenkung des auslenkbaren Betätigungsmittels 108 führt nun dazu, dass sich das in einem Zahnzwischenraum befindliche freie Ende des Betätigungsmittels 108 an einem Zahn abstützt und die bewegliche Vorrichtung 901 in Freilaufrichtung 106 relativ zu dem Rastenelement 103 bewegt. Erfindungsgemäß wird das Rastenelement 103 mit jedem Auslenkvorgang des Betätigungsmittels 108 nur um jeweils eine Raste weiterbewegt. Das heißt, das Betätigungsmittel 108 schiebt das Rastenelement 103 immer nur schrittweise um je eine Raste weiter.

Bei allen zuvor beschriebenen Ausführungsbeispielen kann das Betätigungsmittel 108 auf unterschiedliche Art und Weise ausgelenkt werden. Zum Beispiel kann das Betätigungsmittel 108 thermisch auslenkbar sein. Das heißt, das Betätigungsmittel 108 kann temperaturabhängig seine Form verändern.

Beispielsweise kann das Betätigungsmittel 108 ein thermischer Biegewandler sein. Der thermische Biegewandler kann beispielsweise ein Bimetallstreifen mit unterschiedlichen Wärmeausdehnungskoeffizienten sein. Der thermische Biegewandler 108 kann auch ein sogenannter Bimorph sein. Während der Bimetallstreifen zwei Metalle mit unterschiedlichen Ausdehnungskoeffizienten aufweist, weist der Bimorph allgemein zwei unterschiedliche Materialien auf. Beispielsweise kann der Bimorph einen Metall aufweisenden ersten aktiven Bereich und einen Silizium aufweisenden zweiten aktiven Bereich aufweisen.

Ein thermischer Biegewandler kann beispielsweise einen aktiven Bereich aufweisen, der thermisch verformbar ist. Der thermische Biegewandler ist vorzugsweise in eine erste Richtung temperaturbedingt auslenkbar. Nach dem Abkühlen geht der thermische Biegewandler wieder in seine ursprüngliche Form zurück.

Der thermische Biegewandler kann auch eine Formgedächtnislegierung, eine sogenannte shape memory alloy, aufweisen.

Das Betätigungsmittel 108 kann vorteilhafter Weise ausgebildet sein, um beim Überschreiten und/oder Unterschreiten eines vordefinierten Grenzwerts ausgelenkt zu werden, um mittels dieser Auslenkung das Rastenelement 103 und die Klinke 104 relativ zueinander rastenweise in Freilaufrichtung 106 zu bewegen.

Hierzu soll noch einmal auf die Figuren 2 sowie 3A bis 3F verwiesen werden. Das Betätigungsmittel 108 sei hier beispielsweise thermisch auslenkbar. Das Betätigungsmittel 108, das Rastenelement 103 und die Klinke 104 sind derart zueinander angeordnet, dass das Rastenelement 103 relativ zu der Klinke 104 nur dann um einen Zahn weiterspringt, wenn eine Grenzwerttemperatur überschritten wird. Das heißt, nur wenn diese Grenzwerttemperatur überschritten wird, wird das Betätigungsmittel 108 so weit ausgelenkt, dass es das Zahnrad 103 so weit verschiebt, dass die Klinke 104 in den nächsten Zahnzwischenraum springt. Das heißt, das Betätigungsmittel 108 ist so ausgelegt und bemaßt, dass es bei Überschreiten des Grenzwerts so weit ausgelenkt wird, dass es eine rastenweise Relativbewegung, d.h. eine genau um einen Rastenzwischenraum weitergehende Relativbewegung, zwischen dem Rastenelement 103 und der Klinke 104 ermöglicht.

Wird die Grenzwerttemperatur hingegen nicht erreicht, dann wird das Betätigungsmittel 108 nicht weit genug ausgelenkt, um das Zahnrad 103 um einen Zahn weiterzubewegen.

Wenn beispielsweise eine Temperatur nach dem Überschreiten der Grenzwerttemperatur wieder abkühlt, dann geht das thermisch auslenkbare Betätigungsmittel 108 wieder in seine Ausgangslage zurück. Dann ist das thermische Betätigungsmittel 108 wieder bereit, um erneut bis zum Erreichen der Grenzwerttemperatur ausgelenkt zu werden und das Zahnrad 103 um einen Zahn, d.h. rastenweise, weiterzubewegen.

Je nach Ausführung des Betätigungsmittels 108 kann dies nicht nur beim Überschreiten, sondern auch beim Unterschreiten einer Grenzwerttemperatur der Fall sein.

Je nach Ausführung des Betätigungsmittels 108 kann auch eine andere als eine thermische Kraft das Auslenken des Betätigungsmittels 108 bewirken, sofern die jeweilige Kraft einen vordefinierten Grenzwert unter- bzw. überschreitet.

Mit der erfindungsgemäßen Vorrichtung 100 kann also jedes Mal, wenn ein Grenzwert unter- bzw. überschritten wird, das Rastenelement 103 relativ zu der Klinke 104 um eine Raste 102a, 102b weiterbewegt werden. Somit kann ein Grenzwertzähler mit der erfindungsgemäßen Vorrichtung realisiert werden, der die Anzahl der Grenzwertunter- bzw. Grenzwertüberschreitungen mitzählt.

Ein Ausführungsbeispiel für einen solchen Grenzwertzähler wird in Form eines Sterilisationszyklenzählers vorgeschlagen, bei dem das Betätigungsmittel 108 bei jedem Sterilisationsvorgang das Rastenelement 103 und die Klinke 104 in Freilaufrichtung relativ zueinander um jeweils eine Raste 102a, 102b verschiebt.

Ein Beispiel für einen temperaturbehafteten Prozess stellt die Dampfsterilisation (Autoklavierung) bei der Aufbereitung von wiederverwendbaren Instrumenten und Komponenten im klinischen Umfeld dar. Im typischen Prozessverlauf der Dampfsterilisation wird, je nach Verfahren, einmalig ein maximales Temperaturniveau von 121 °C bzw. 134 °C erreicht, wie in Figur 8 skizziert. Hier stellt die Kurve 1010 den Temperaturverlauf und die Kurve 1020 den Druckverlauf über die Zeit dar.

Das Erreichen und Feststellen des zugehörigen Temperaturgrenzwertes kann folglich einerseits als Charakteristikum für einen erfolgreich durchgeführten Sterilisationszyklus gewertet werden. Andererseits ermöglicht ein "Aufzählen" der Einzelzyklen die Erfassung der Gesamtzahl durchlaufener Sterilisationszyklen, z.B. im Hinblick auf eine maximal zulässige Anzahl von Dampfsterilisationen während des Lebenszyklus eines Instruments.

Beispielsweise ist die Dampfsterilisation (Erhitzen im Autoklaven) das Standardverfahren in den meisten Labors und Krankenhäusern, um Materialien und Gegenstände von lebenden Mikroorganismen einschließlich ihrer Ruhestadien (z. B. Sporen) zu befreien.

Dabei wird beispielsweise das Sterilisier- oder Füllgut:
- 20 Minuten auf 121 °C bei 2 bar Druck in Wasserdampf erhitzt, oder
- 5 Minuten auf 134 °C bei 3 bar, oder
- 18 Minuten auf 134 °C bei 3 bar (Zerstörung von Prionen)

Die erfindungsgemäße Vorrichtung weist nutzerspezifische Vorteile auf, d.h. sie hat Vorteile für
a) den Gerätehersteller:
   - Anzahl Sterilisationszyklen je Gerät, u.a. wegen Gewährleistung/Benutzung über das spezifizierte Maß hinaus
   - Gewährleistungsfrage insbesondere bei sehr hochwertigen Geräten
b) den Gerätenutzer:
   - Überprüfung ob und wie oft Gerät sterilisiert wurde / Logistik

Somit können manche Ausführungsbeispiele der erfindungsgemäßen Vorrichtung wie folgt zusammengefasst werden:
1. Thermisch getriggerter Aktuator bewegt mikromechanische Struktur (Zahnrad) bei kritischer Temperatur
2. Mechanische Bewegung um "eine Raste" (= 1 Zyklus) verändert Kapazität
3. Veränderung der Kapazität verändert Charakteristik eines passiven RFID-Schwingkreises
4. Auslesen des veränderten Schwingkreises (= Zählerstand) mit aktivem RFID-Reader

Ausführungsbeispiele der Erfindung stellen also einen energieautarken MEMS-Sterilisationszyklenzähler bereit, der wie ein passives RFID-Tag ausgelesen werden kann.

Die Erfindung soll nachfolgend nochmals in anderen Worten beschrieben werden:
Ein Ausführungsbeispiel der Erfindung sieht eine Vorrichtung vor, z.B. in Form eines Mikrosystems (MEMS), die autonom, d.h. ohne interne oder externe elektrische Energiezuführung das Erreichen definierter Temperaturgrenzwerte zählen und speichern kann sowie ein elektrisches Auslesen der Anzahl erreichter Temperaturgrenzwertereignisse zu einem späteren Zeitpunkt ermöglicht.

So ist in Figur 2 als ein Beispiel einer erfindungsgemäßen Vorrichtung ein MEMS Temperaturgrenzwertzähler schematisch dargestellt, der unter anderem die folgenden Komponenten aufweist:
- ein Substrat 210, z.B. einem Wafer aus Silizium;
- eine mit Zähnen behafteten beweglichen Struktur (Rastenelement) 103 auf dem stationären Substrat 210, z.B. einem freidrehenden Zahnrad, das in Siliziumoberflächen- oder Bulkmikromechanik hergestellt wurde;
- eine Klinke 104, die eine Bewegung der Zahnstruktur 103 in nur in eine Richtung (Freilaufrichtung) 106 erlaubt, z.B. einer Sperrklinke, die ebenfalls in Siliziumoberflächen- oder Bulkmikromechanik hergestellt wurde;
- ein Antriebsmechanismus (Betätigungsmittel) 108, der mit seiner thermisch induzierten Bewegung direkt oder mit Hilfe weiterer Elemente, z.B. einer Umlenkung, die Zahnstruktur 103 bewegt und sie dabei entgegengesetzt der durch die Klinke 104 gesperrten Richtung bewegt, z.B. einem thermischen Biegewandler der aus zwei Materialen mit unterschiedlichen Temperaturausdehnungskoeffizienten aufgebaut ist;
- ein elektrisches Bauelement 109, das sowohl mit dem Substrat 210 als auch der beweglichen Zahnstruktur 103 derart interagiert, dass eine Bewegung der beiden Teile 210, 103 relativ zueinander eine eindeutige Veränderung der elektrischen Eigenschaften des Bauelements 109 bewirkt, z.B. wenn das stationäre Substrat 210 eine Elektrode ausbildet und die beweglichen Zahnstruktur 103 die zweite Elektrode eines Kondensators, so dass eine Verschiebung der beiden Elektroden relativ zueinander die Kapazität des elektrischen Kondensators verändern; und
- eine elektronische Schnittstelle 209, die nicht notwendigerweise Teil des MEMS-Elements selbst sein muss und ein Auslesen der Veränderung des elektrischen Bauelements 109 ermöglicht, z.B. wenn das elektrische Bauelement 109 direkt das verstellbare Glied eines RFID Schwingkreises oder einer aufwendigeren Elektronik darstellt.

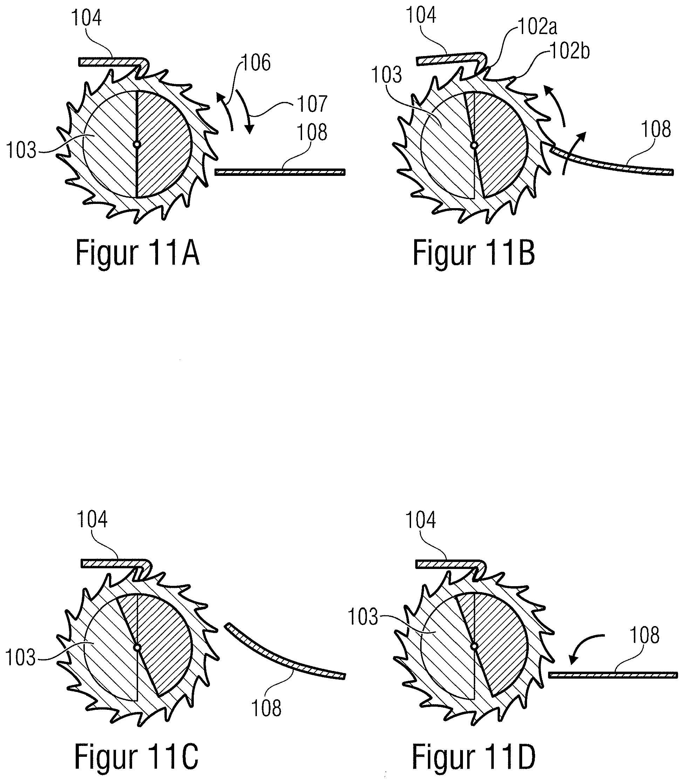

Die beispielhafte Funktionsweise des MEMS Temperaturgrenzwertzähler ist in den Figuren 11A bis 11D nochmals schematisch dargestellt. Bei Über- bzw. Unterschreiten der kritischen Temperaturgrenze vollzieht der Antriebsmechanismus (Betätigungsmittel) 108 eine thermisch induzierte Bewegung, z.B. von der Anfangsstellung (Figur 11A) zur Endstellung (Figur 11C), in deren Verlauf er die Zahnstruktur (Rastenelement) 103 bewegt und sie entgegengesetzt der durch die Klinke 104 gesperrten Richtung 107 um einen Schritt (Zahn) 102a, 102b bewegt. Die Zahnstruktur 103 gleitet dabei an der Klinke 104 vorbei.

Solange die Temperaturgrenze nicht wieder unter- bzw. überschritten wird, bleibt dabei der Antriebsmechanismus 108 in der Endstellung (Figur 11C). Wird jedoch die Temperaturgrenze wieder unter- bzw. überschritten, dann vollzieht der Mechanismus wieder eine Bewegung von der Endstellung (Figur 11C) zur Anfangsstellung (Figur 11D). Im Verlauf dieser Rückbewegung kommt der Antriebsmechanismus 108 wieder in Kontakt mit der Zahnstruktur 103. Allerdings verhindert die Klinke 104, dass sich die Zahnstruktur 103 wieder in die ursprüngliche Position zurückdreht, so dass der Antriebsmechanismus 108 über die Zahnstruktur 103 hinweggleiten muss und kann.

Bei jedem Über- bzw. Unterschreiten der Temperaturgrenze sowie nachfolgendem Unter- bzw. Überschreiten der Temperaturgrenze wiederholt sich die beschriebene Abfolge wobei jeweils die Zahnstruktur 103 um einen Schritt (Zahn) 102a, 102b weiterbewegt wird und sich somit die Lage der Zahnstruktur 103 relativ zum Substrat 210 ändert.

Da sich mit dieser Bewegung auch die Eigenschaft des elektrischen Bauelements 109 eindeutig ändert, ist die Eigenschaft des elektrischen Bauelements 109 charakteristisch für die Lage der Zahnstruktur 103 relativ zum Substrat 210 und somit für die Anzahl der bis dato vollzogenen Schritte bzw. weitergesprungenen Zähne, was der Anzahl stattgefundener Temperaturgrenzwertereignisse entspricht. Die bis jetzt genannten Funktionen des MEMS-Elements laufen energieautonom ab, d.h. es wird keine Zuführung elektrischer Energie benötigt.

Mit Hilfe der mit Bezug auf Figur 2 erläuterten elektronischen Schnittstelle 209 kann ein Nutzer jederzeit die Eigenschaften des elektrischen Bauelements 109 und somit die bis dato stattgefundenen Temperaturgrenzwertereignisse auslesen. Hierzu bestehen verschiedene Möglichkeiten von denen drei nachfolgend beispielhaft erläutert werden:
1. Im einfachsten Fall erfolgt das direkte Auslesen der Eigenschaft des elektrischen Bauelements 109 durch drahtgebundene Kontaktierung mit einem entsprechenden Messgerät, was eine Bestimmung der stattgefundenen Temperaturgrenzwertereignisse ermöglicht.
2. Entspricht das elektrische Bauelement 109 einem typischen Element eines Schwingkreises (Kondensator, Spule, Widerstand), z.B. einem veränderlichen Kondensator, und bildet dieser zusammen mit einer Spulenstruktur 206 einen Schwingkreis 207, so ändert eine Veränderung der Kapazität auch die Schwingcharakteristik des Schwingkreises (dargestellt in Figur 2). Der so entstehende passive Transponder eines RFID-Systems (engl. radio-frequency identification) kann mit einem entsprechenden Lesegerät drahtlos ausgelesen werden und so wiederum die stattgefundenen Temperaturgrenzwertereignisse bestimmt werden.
3. Ist das elektrische Bauelement 109 Teil eines elektronischen Schaltkreises, der wiederum Teil eines RFID-Transpondersystems ist, so kann mit Hilfe eines entsprechenden Lesegeräts elektrische Energie drahtlos in den Schaltkreis eingekoppelt werden und zum Ausführen von Funktionen des elektronischen Schaltkreises verwendet werden, z.B. zu Signalverstärkungen, -auswertungen, und weiteren Sendeaufgaben.

Wie bereits erwähnt, kann das erfindungsgemäße Konzept zum Ermitteln unterschiedlicher Grenzwertereignisse verwendet werden. Eines von mehreren Beispielen hierfür wäre die Verwendung als Sterilisationszyklenzähler. Hierbei wird beispielsweise Operationsbesteck nach seiner Verwendung mittels heißem Dampf sterilisiert.

Generell weist die erfindungsgemäße Vorrichtung unter anderem die folgenden Vorteile auf:
- Energieautonome Feststellung des Temperaturpeaks, keine elektrische Energiequelle notwendig
- Elektrische Auslesbarkeit des Zählerstands durch passives RFID-Konzept
- MEMS-Technik, dadurch kleines, vielseitig einsetzbares Bauteil
- In größeren Stückzahlen wohl kostengünstiger als FWT-Variante
- Siehe Transfer FWT Drehratensensor nach MEMS Drehratensensor
- Fälschungssicher im Hinblick, dass nie weniger Zyklen stattgefunden haben können als registriert wurden

Mögliche technische Merkmale:
- Biege-Aktuator in Waferebene
- Freidrehende Mikrostruktur mit Rastmechanismen
- Kombination mechanisch verstellbarer Kondensator mit passivem RFID Schwingkreis Weitere Umsetzungsideen:
- Spule anstelle Kondensator verstellen: Spiralfeder, zwei Spiralfedern ineinander

Natürlich sind die oben genannten Beispiele nicht abschließend bzw. einschränkend. Weitere Ausführungsbeispiele können geschaffen werden, die sich wie folgt unterteilen lassen:
Anhand Aufbau Zahnstruktur:
   a) Zahnstruktur
      a. freidrehend (Figur 2)
      b. über Festkörpergelenk/Feder auslenkbar
      c. feststehend
   b) Bewegliche Zahnstruktur
      a. radiale Innen- bzw. Außenverzahnung (Figur 2)
      b. lineare Innen- bzw. Außenverzahnung (Zahnschiene)
Anhand Funktionsablauf:
   a) Zahnstruktur passiv + Raste passiv → Antrieb bewegt Zahnrad (Figuren 2, 3A bis 3F)
   b) Zahnstruktur vorgespannt → Antrieb bewegt Rastmechanismus (Prinzip Ankerhemmung - Figur 6), d.h. Raste und Antrieb bilden eine Einheit
   c) Zahnstruktur feststehend, Antrieb mit Raste "zieht" sich über Zahnstruktur
Anhand Änderung elektrisches Bauelement:
   a) Kondensator
   b) Spule
   c) Widerstand (Ohm'scher bzw. piezoresistiver)
   d) Elektrooptisches Element

Außerdem werden nachfolgend Ausführungsbeispiele für das Betätigungsmittel 108 vorgeschlagen:
- Thermische Biegewandler (Bimorph-Aktuatoren), die aus zwei Materialien mit verschiedenen Wärmeausdehnungskoeffizienten aufgebaut sein können und sich insbesondere parallel oder in der Waferebene bewegen können, wobei letzteres im vorliegenden Fall technologisch sehr interessant ist.
- Formgedächtnislegierungen ("shape memory alloys")
- Aktuatoren, die die thermische Festkörperausdehnung direkt ausnutzen und dann ggf. zur Hubvergrößerung noch übersetzt werden (z.B. mit Winkelstrukturen, engl. "Chevron-Structures").
- Ausnutzung der Volumenänderung einer Flüssigkeit
- Ausnutzung der Volumenänderung beim thermischen Phasenwechsel

Bei der erfindungsgemäßen Vorrichtung 100 kann, neben der bereits erwähnten Temperatur, auch eine Beschleunigung, ein Druck, etc. als mögliches Grenzwertereignis berücksichtigt sein, weshalb die erfindungsgemäße Vorrichtung auch als ein Grenzwertgeber bzw. als eine Grenzwertermittlungsvorrichtung bezeichnet werden kann. Eine Eigenschaft, die die erfindungsgemäße Vorrichtung 100 aufweist, ist die Möglichkeit, dass beim hier beschriebenen Ansatz sichergestellt sein könnte, dass pro Grenzwertereignis nur um eine Raste bzw. um einen Zahn weitergesprungen wird.

Weitere denkbare Ausführungsbeispiele einer erfindungsgemäßen Vorrichtung können daher beispielsweise sein:
Autonomer MEMS-Grenzwertzähler (z.B. Temperaturgrenzwertzähler) aufweisend:
   - ein Substrat 210 (Substrat schließt - bei Vorstellung eines geschlossenen Gehäuses - sowohl "Boden" als auch "Deckel" und "Seiten" ein)
   - eine mit Zähnen 102a, 102b behaftete bewegliche Struktur 103
   - einen thermisch getriebenen Antriebsmechanismus 108
   - ein auslesbares elektrisches Bauelement 109, das sowohl mit der beweglichen Struktur 103 als auch dem Substrat 210 interagiert,
wobei bei Über- bzw. Unterschreiten eines Temperaturgrenzwertes der Antriebsmechanismus 108 die mit Zähnen 102a, 102b behaftete Struktur 103 bewegt und sich somit die elektrischen Eigenschaften des elektrischen Bauelements 109 ändern.

Weitere denkbare Ausführungsbeispiele sind im Folgenden stichpunktartig aufgeführt:
- Der Temperaturgrenzwertzähler kann in Siliziumtechnologie hergestellt sein
- Ein Mechanismus kann die Rückbewegung der beweglichen Struktur 103 bei Rückbewegung des Antriebsmechanismus 108 verhindern
- Die mit Zähnen 102a, 102b behaftete Struktur 103 kann keine feste Verbindung zum Substrat 210 aufweisen
   ∘ Bei der mit Zähnen 102a, 102b behafteten beweglichen Struktur 103 kann es sich um ein freidrehendes Zahnrad handeln
   ∘ Bei der mit Zähnen 102a, 102b behafteten Struktur 103 kann es sich um ein linear verschiebbares Element handeln
- Die mit Zähnen 102a, 102b behaftete Struktur 103 kann über ein Festkörpergelenk mit dem Substrat 210 verbunden sein
- Bei dem elektrischen Bauelement 109 kann es sich um ein verstellbares Glied eines RFID-Schwingkreises 207 handeln
   ∘ Es kann sich um einen passiven RFID-Schwingkreis 207 handeln
   ∘ Es kann sich bei dem elektrischen Bauelement 109 um
      ▪ einen Kondensator handeln
      ▪ einen Widerstand handeln
      ▪ um eine Spule handeln
- Das elektrische Bauelement 109 kann mit einer Elektronik verbunden sein
   ∘ Die Elektronik wiederum kann Teil eines RFID Schwingkreises 207 sein
   ∘ Die Elektronik kann die Veränderung des elektrischen Bauelements 109 verstärken
   ∘ Die Elektronik kann über den RFID-Schwingkreis 207 mit Energie versorgt werden
- Der Antriebsmechanismus 108 kann ein Biegewandler sein
   ∘ Es kann sich um einen thermischen Bimorph-Aktuator handeln
   ∘ Es kann sich um thermische Festkörperausdehnung handeln
- Der Antriebsmechanismus 108 kann mittels Über- oder Untersetzung auf das mit Zähnen 102a, 102b behaftete Element 103 wirken
   o Die Über- bzw. Untersetzung kann eine Bewegung parallel zur Substratoberfläche in eine Bewegung in der Substratebene umsetzen
- Der Temperaturgrenzwertzähler kann ein Überschreiten eines definierten Temperaturgrenzwerts detektieren
   ∘ Der Temperaturgrenzwertzähler kann als Sterilisationszyklenzähler eingesetzt werden

### Quellen:

[1] X.-Q. Sun, S. Zhou, and W. N. Carr, "A surface micromachined latching accelerometer", International Conference on Solid State Sensors and Actuators 1997, 1189-1192.
[2] H. Mehner, C. Weise, S. Schwebke, .S. Hampl, M. Hoffmann, "A passive microsystem for detecting multiple acceleration events beyond a threshold", Microelectronic Engineering 145 (2015), 104-111.

## Patentansprüche

1. Vorrichtung (100) mit
einem angeordneten Rastmechanismus (101) mit einem mindestens zwei Rasten (102a, 102b) aufweisenden Rastenelement (103) und einer Klinke (104), die ausgebildet ist, um in einen Rastenzwischenraum (105) zwischen zwei Rasten (102a, 102b) einzugreifen,
wobei das Rastenelement (103) in einer Freilaufrichtung (106) relativ zu der Klinke (104) bewegbar ist und eine Bewegung des Rastenelements (103) relativ zu der Klinke (104) in einer Sperrrichtung (107) mittels der Klinke (104) blockierbar ist,
einem auslenkbaren Betätigungsmittel (108), das ausgebildet ist, um mittels einer Auslenkung das Rastenelement (103) und die Klinke (104) relativ zueinander rastenweise in Freilaufrichtung (106) zu bewegen, und
einem elektrischen Bauelement (109), das ausgebildet ist, um seine elektrische Eigenschaft in Abhängigkeit von der rastenweisen Bewegung des Rastenelements (103) relativ zu der Klinke (104) zu verändern,
**dadurch gekennzeichnet, dass**
der Rastmechanismus (101) auf einem Substrat (210) angeordnet und als ein MEMS-Mikrosystem ausgeführt ist.

2. Vorrichtung (100) nach einem der vorhergehenden Ansprüche,
wobei das elektrische Bauelement (109) ein Kondensator ist, wobei eine erste Kondensatorplatte (201) an dem Substrat (210) und eine zweite Kondensatorplatte (202) an dem Rastenelement (103) und/oder an der Klinke (104) bereitgestellt ist, und wobei sich bei einer rastenweisen Bewegung des Rastenelements (103) und/oder der Klinke (104) relativ zu dem Substrat (210) die Ausrichtung der Kondensatorplatten (201,202) relativ zueinander verändert, wobei sich die Kondensatorkapazität ändert.

3. Vorrichtung (100) nach Anspruch 1 oder 2,
wobei eine Auslenkung des Betätigungsmittels (108) in einer Ebene horizontal zu der Substratebene geschieht, oder
wobei eine Auslenkung des Betätigungsmittels (108) in einer Ebene senkrecht zu der Substratebene geschieht, und die Vorrichtung (100) ferner eine Umlenkvorrichtung (700) aufweist mittels der die senkrecht zu der Substratebene gerichtete Auslenkbewegung des Betätigungsmittels (108) in eine horizontal zur Substratebene gerichtete Bewegung umlenkbar ist.

4. Vorrichtung (100) nach einem der vorhergehenden Ansprüche,
wobei die Rasten (102a, 102b) entlang des Rastenelements (103) in Freilaufrichtung (106) hintereinander angeordnet sind, sodass die Klinke (104) bei der rastenweisen Bewegung nacheinander von einem Rastenzwischenraum (105a) in den jeweils nächsten benachbarten Rastenzwischenraum (105b) eingreift.

5. Vorrichtung (100) nach einem der vorhergehenden Ansprüche,
wobei das Rastenelement (103) ein frei drehbares Zahnrad ist, bei dem die Rasten (102a, 102b) in Form einer radial außenseitig oder radial innenseitig an dem Zahnrad (103) angeordneten Verzahnung ausgebildet sind, oder
wobei das Rastenelement (103) eine relativ zu der Klinke (104) bewegbare Zahnstange ist, bei der die Rasten (102a, 102b) in Form einer an der Zahnstange (103) angeordneten Verzahnung ausgebildet sind.

6. Vorrichtung (100) nach einem der vorhergehenden Ansprüche,
wobei das Betätigungsmittel (108) das Rastenelement (103) betätigt, um das Rastenelement (103) relativ zu der Klinke (104) in Freilaufrichtung (106) rastenweise um jeweils eine Raste (102a, 102b) weiterzubewegen.

7. Vorrichtung (100) nach Anspruch 6,
wobei das Betätigungsmittel (108) an einer Raste (102a, 102b) des Rastenelements (103) angreift, um das Rastenelement (103) relativ zu der Klinke (104) rastenweise weiterzubewegen.

8. Vorrichtung (100) nach einem der vorhergehenden Ansprüche,
wobei das Rastenelement (103) mittels eines Spannelements (501) vorgespannt ist und das Betätigungsmittel (108) die Klinke (104) betätigt, wobei sich bei einer den Eingriff in einen Rastenzwischenraum (105a) lösenden Bewegung der Klinke (104) das vorgespannte Rastenelement (103) aufgrund der Vorspannung jeweils um eine Raste (102a, 102b) weiterbewegt bevor die Klinke (104) wieder in einen benachbarten nächsten Rastenzwischenraum (105b) des Rastenelements (103) eingreift.

9. Vorrichtung (100) nach einem der vorhergehenden Ansprüche,
wobei das Betätigungsmittel (108) thermisch oder mechanisch oder elektrisch auslenkbar ist.

10. Vorrichtung (100) nach Anspruch 9,
wobei das Betätigungsmittel (108) ein thermischer Biegewandler ist, oder wobei das Betätigungsmittel (108) eine Formgedächtnislegierung aufweist.

11. Vorrichtung (100) nach einem der vorhergehenden Ansprüche,
wobei das Betätigungsmittel (108) ausgebildet ist, um beim Überschreiten und/oder Unterschreiten eines vordefinierten Grenzwerts ausgelenkt zu werden, um mittels dieser Auslenkung das Rastenelement (103) und die Klinke (104) relativ zueinander rastenweise in Freilaufrichtung (106) zu bewegen.

12. Vorrichtung (100) nach einem der vorhergehenden Ansprüche,
wobei das elektrische Bauelement (109) ein Widerstand oder eine Spule oder ein elektrooptisches Element ist.

13. Vorrichtung (100) nach einem der vorhergehenden Ansprüche,
wobei die Vorrichtung (100) einen RFID-Schwingkreis (207) aufweist und das elektrische Bauelement (109) ein verstellbares Glied des RFID-Schwingkreises (207) ist.

14. Vorrichtung (100) nach einem der vorhergehenden Ansprüche,
wobei das elektrische Bauelement (109) zwischen dem Rastmechanismus (101) und dem Substrat (210) angeordnet ist.

15. Vorrichtung (100) nach einem der vorhergehenden Ansprüche,
wobei die Vorrichtung (100) als ein Sterilisationszyklenzähler ausgebildet ist, bei dem das Betätigungsmittel (108) bei jedem Sterilisationsvorgang das Rastenelement (103) und die Klinke (104) in Freilaufrichtung (106) relativ zueinander um jeweils eine Raste (102a, 102b) verschiebt.

## Claims

1. Device (100) comprising
a latching mechanism (101) comprising a catch element (103) having at least two catches (102a, 102b), and a pawl (104) configured to engage in a catch interstice (105) between two catches (102a, 102b),
wherein the catch element (103) is movable in relation to the pawl (104) in a freewheeling direction (106) and wherein a movement of the catch element (103) in relation to the pawl (104) in a blocking direction (107) may be blocked by means of the pawl (104),
deflectable actuation means (108) configured to move the catch element (103) and the pawl (104) relative to each other on a catch-by-catch basis in the freewheeling direction (106) by means of a deflection, and
an electric component (109) configured to change its electric property as a function of the catch-wise movement of the catch element (103) in relation to the pawl (104),
**characterized in that**
the latching mechanism (101) is arranged on a substrate and is configured as a MEMS microsystem.

2. Device (100) as claimed in any of the previous claims,
wherein the electric component (109) is a capacitor, wherein a first capacitor plate (201) is provided at the substrate (210) and a second capacitor plate (202) is provided at the catch element (103) and/or at the pawl (104), and wherein upon catch-wise movement of the catch element (103) and/or of the pawl (104) in relation to the substrate (210), the mutual alignment of the capacitor plates (201, 202) in relation to each other changes, wherein the capacitance of the capacitor changes.

3. Device (100) as claimed in claims 1 or 2,
wherein deflection of the actuation means (108) takes place within a plane that is horizontal to the substrate plane, or
wherein deflection of the actuation means (108) takes place within a plane that is perpendicular to the substrate plane, and the device (100) further comprises a diverting device (700) by means of which the deflection movement of the actuation means (108), which is directed perpendicularly to the substrate plane, may be diverted to a movement that is directed horizontally to the substrate plane.

4. Device (100) as claimed in any of the previous claims,
wherein the catches (102a, 102b) are arranged one behind the other along the catch element (103) in the freewheeling direction (106), so that the pawl (104) will consecutively engage, in the catch-wise movement, from one catch interstice (105a) into the respectively next adjacent catch interstice (105b).

5. Device (100) as claimed in any of the previous claims,
wherein the catch element (103) is a freely rotatable gearwheel wherein the catches (102a, 102b) are configured in the form of a toothing arranged radially on the outside or on the inside of the gearwheel (103) or
wherein the catch element (103) is a rack that is movable in relation to the pawl (104) and wherein the catches (102a, 102b) are configured in the form of a toothing arranged on the rack (103).

6. Device (100) as claimed in any of the previous claims,
wherein the actuation means (108) actuates the catch element (103) to move the catch element (103) further by one catch (102a, 102b), respectively, on a catch-by-catch basis in relation to the pawl (104) in the freewheeling direction (106).

7. Device (100) as claimed in claim 6,
wherein the actuation means (108) acts upon a catch (102a, 102b) of the catch element (103) so as to move the catch element (103) further, on a catch-by-catch basis, in relation to the pawl (104).

8. Device (100) as claimed in any of the previous claims,
wherein the catch element (103) is biased by means of a tensioning element (501) and wherein the actuation means (108) actuates the pawl (104), wherein upon a movement of the pawl (104), which releases the engagement with a catch interstice (105a), the biased catch element (103) is moved forward by one catch (102a, 102b) in each case due to the bias before the pawl (104) engages in an adjacent next catch interstice (105b) of the catch element (103) again.

9. Device (100) as claimed in any of the previous claims,
wherein the actuation means (108) is thermally or mechanically or electrically deflectable.

10. Device (100) as claimed in claim 9,
wherein the actuation means (108) is a thermal bending transducer, or wherein the actuation means (108) comprises a shape memory alloy.

11. Device (100) as claimed in any of the previous claims,
wherein the actuation means (108) is configured to be deflected once a predefined threshold value is exceeded or fallen below so as to move, by means of said deflection, the catch element (103) and the pawl (104) in relation to each other on a catch-by-catch basis in the freewheeling direction (106).

12. Device (100) as claimed in any of the previous claims,
wherein the electric component (109) is a resistor or a coil or an electro-optical element.

13. Device (100) as claimed in any of the previous claims,
wherein the device (100) comprises an RFID resonant circuit (207) and the electric component (109) is an adjustable member of the RFID resonant circuit (207).

14. Device (100) as claimed in any of the previous claims,
wherein the electric component (109) is arranged between the latching mechanism (101) and the substrate (210).

15. Device (100) as claimed in any of the previous claims,
the device (100) being configured as a counter of sterilization cycles, wherein the actuation means (108) shifts, with each sterilization process, the catch element (103) and the pawl (104) by one catch (102a, 102b), respectively, in relation to each other in the freewheeling direction (106).

## Revendications

1. Dispositif (100) avec
un mécanisme à crans (101) avec un élément à crans (103) présentant au moins deux crans (102a, 102b) et un cliquet (104) qui est conçu pour s'engager dans un interstice de cran (105) entre deux crans (102a, 102b),
dans lequel l'élément à crans (103) est déplaçable par rapport au cliquet (104) dans une direction en roue libre (106) et un déplacement de l'élément à crans (103) par rapport au cliquet (104) dans une direction de blocage (107) peut être bloqué au moyen du cliquet (104),
un moyen d'actionnement pouvant être dévié (108) qui est conçu pour déplacer, au moyen d'une déviation, l'élément à crans (103) et le cliquet (104) l'un par rapport à l'autre, cran par cran, dans la direction en roue libre (106), et
un composant électrique (109) qui est conçu pour modifier sa propriété électrique en fonction du déplacement cran par cran de l'élément à crans (103) par rapport au cliquet (104),
**caractérisé par le fait que**
le mécanisme à crans (101) est disposé sur un substrat (210) et est conçu comme un microsystème MEMS.

2. Dispositif (100) selon l'une des revendications précédentes,
dans lequel le composant électrique (109) est un condensateur, dans lequel une première plaque de condensateur (201) est prévue sur le substrat (210) et une deuxième plaque de condensateur (202) est prévue sur l'élément à crans (103) et/ou sur le cliquet (104), et dans lequel, lors d'un déplacement cran par cran de l'élément à crans (103) et/ou du cliquet (104) par rapport au substrat (210), l'orientation des plaques de condensateur (201, 202) l'une par rapport à l'autre change, la capacité du condensateur changeant.

3. Dispositif (100) selon la revendication 1 ou 2,
dans lequel une déviation du moyen d'actionnement (108) a lieu dans un plan horizontal par rapport au plan du substrat, ou
dans lequel une déviation du moyen d'actionnement (108) a lieu dans un plan perpendiculaire au plan du substrat, et le dispositif (100) présente par ailleurs un dispositif de déviation (700) au moyen duquel le déplacement de déviation du moyen d'actionnement (108) orienté perpendiculairement au plan du substrat peut être dévié à un déplacement orienté horizontalement par rapport au plan du substrat.

4. Dispositif (100) selon l'une des revendications précédentes,
dans lequel les crans (102a, 102b) sont disposés l'un derrière l'autre le long de l'élément à crans (103) dans la direction en roue libre (106), de sorte que le cliquet (104) s'engage, lors du déplacement cran par cran, successivement d'un interstice de cran (105a) dans l'interstice de cran respectivement suivant (105b).

5. Dispositif (100) selon l'une des revendications précédentes,
dans lequel l'élément à crans (103) est une roue d'engrenage pouvant tourner librement, où les crans (102a, 102b) sont conçus en forme de denture disposée du côté radialement extérieur ou du côté radialement intérieur sur la roue d'engrenage (103), ou
dans lequel l'élément à crans (103) est une crémaillère déplaçable par rapport au cliquet (104), où les crans (102a, 102b) sont conçus en forme d'une denture disposée sur la crémaillère (103).

6. Dispositif (100) selon l'une des revendications précédentes,
dans lequel le moyen d'actionnement (108) actionne l'élément à crans (103) pour déplacer l'élément à crans (103), cran par cran, chaque fois d'un cran (102a, 102b), par rapport au cliquet (104) dans la direction en roue libre (106).

7. Dispositif (100) selon la revendication 6,
dans lequel le moyen d'actionnement (108) vient en prise avec un cran (102a, 102b) de l'élément à cran (103) pour déplacer l'élément à crans (103), cran par cran, par rapport au cliquet (104).

8. Dispositif (100) selon l'une des revendications précédentes,
dans lequel l'élément à crans (103) est précontraint au moyen d'un élément de tension (501) et le moyen d'actionnement (108) actionne le cliquet (104), dans lequel, lors d'un mouvement de dégagement de la venue en prise du cliquet (104) dans un interstice de cran (105a), l'élément à crans (103) précontraint se déplace, du fait de la précontrainte, chaque fois d'un cran (102a, 102b) avant que le cliquet (104) ne s'engage à nouveau dans un interstice de cran suivant adjacent (105b) de l'élément à crans (103).

9. Dispositif (100) selon l'une des revendications précédentes,
dans lequel le moyen d'actionnement (108) peut être dévié de manière thermique ou mécanique ou électrique.

10. Dispositif (100) selon la revendication 9,
dans lequel le moyen d'actionnement (108) est un transducteur thermique à flexion, ou dans lequel le moyen d'actionnement (108) présente un alliage à mémoire de forme.

11. Dispositif (100) selon l'une des revendications précédentes,
dans lequel le moyen d'actionnement (108) est conçu pour être dévié au cas où est excédée et/ou il est descendu au-dessous d'une valeur limite prédéfinie, pour déplacer, au moyen de cette déviation, l'élément à crans (103) et le cliquet (104), cran par cran, l'un par rapport à l'autre dans la direction en roue libre (106).

12. Dispositif (100) selon l'une des revendications précédentes,
dans lequel le composant électrique (109) est une résistance ou une bobine ou un élément électro-optique.

13. Dispositif (100) selon l'une des revendications précédentes,
dans lequel le dispositif (100) présente un circuit résonnant RFID (207) et le composant électrique (109) est un élément réglable du circuit résonnant RFID (207).

14. Dispositif (100) selon l'une des revendications précédentes,
dans lequel le composant électrique (109) est disposé entre le mécanisme à crans (101) et le substrat (210).

15. Dispositif (100) selon l'une des revendications précédentes,
dans lequel le dispositif (100) est conçu comme un compteur de cycles de stérilisation, dans lequel le moyen d'actionnement (108) déplace, à chaque opération de stérilisation, l'élément à crans (103) et le cliquet (104) dans la direction en roue libre (106) l'un par rapport à l'autre de chaque fois un cran (102a, 102b).
